# EUROPEAN PATENT SPECIFICATION

(11) **EP 3 280 496 B1**
(45) Date of publication and mention of the grant of the patent: **20.07.2022**
(21) Application number: 16722724.8
(22) Date of filing: 06.04.2016
(51) Int. Cl.: A61Q 5/00, A61Q 11/00, A61Q 13/00, A61Q 15/00, A61Q 19/00, A61K 8/89, C11D 3/37, C11D 3/43, C11D 3/50

(54) **EXTENDED LONGEVITY FRAGRANCE DELIVERY COMPOSITION**
DUFTFREISETZUNGSZUSAMMENSETZUNG MIT VERLÄNGERTER LANGLEBIGKEIT
COMPOSITION DE DISTRIBUTION DE PARFUM À DURÉE PROLONGÉE

(30) Priority: 09.04.2015 IN 1482MU2015
(43) Date of publication of application: 14.02.2018
(73) Proprietor: Momentive Performance Materials Inc., Waterford, NY 12188 (US)
(72) Inventor: SUN, Kunshan, Millwood, NY 10546 (US); DUSSAUD, Anne, Tarrytown, NY 10591 (US); FALK, Benjamin, Yorktown Heights, NY 10598 (US); PHUKAN, Monjit, Bangalore 560075 (IN); STASIAK, Nicholas, Putnam Valley, NY 10579 (US); GONZALEZ, Sigfredo, Danbury, CT 06811 (US)
(74) Representative: Laufhütte, Dieter
(86) International application number: PCT/US2016/026160
(87) International publication number: WO 2016/164421

(56) References cited:
- EP-A2- 0 982 022
- WO-A1-2010/109462
- WO-A2-01/91712
- WO-A2-2013/103496

## Description

### FIELD OF THE INVENTION

This invention is directed to fragrance-containing compositions possessing enhanced fragrance longevity. More particularly, the invention provides fragrance-containing compositions, e.g., personal care, home care and fabric care compositions, containing an acid functional silicone compound as a fragrance longevity enhancing agent.

### BACKGROUND OF THE INVENTION

Fragrance compositions are ubiquitous components of personal care, home care and fabric care products. Numerous materials, hereinafter referred to as fixatives, have been proposed over the years for extending the longevity or retention of the fragrance component of such compositions. Even relatively slight improvements in fragrance retention are considered to be highly desirable in as much as a product's pleasing scent plays an important role in influencing consumers' acceptance and preferences.

Prior art document WO 2010/109462 A1 discloses perfumes having extended longevity obtained by enriching the perfumes with a mixture of salts and minerals. Prior art document WO 2013/103496 A2 discloses silicone ionomer compositions for use in various applications, e.g. personal care applications.

### SUMMARY OF THE INVENTION

It has now been discovered that an acid-functional silicone of a particular class can function as a fixative for the fragrance component of fragrance-containing compositions such as the aforementioned personal care, fabric care and home care products.

In accordance with the present invention, there is provided a fragrance delivery composition in the form of an aqueous emulsion or suspension, a non-aqueous emulsion or suspension, or an organic solvent solution, the composition comprising:

(a) a fragrance longevity-enhancing amount of 0.01 wt% to 60 wt% of neutralized acid-functional silicone of the general formula (I):

   MₐMⁱ_{b}D_{c}Dⁱ_{d}TₑTⁱ_{f}Q_{g} (I)

   wherein
   M = R¹R²S³SiO_{1/2};
   Mⁱ = R⁴R⁵RⁱSiO_{1/2};
   D = R⁶R⁷SiO_{2/2};
   Dⁱ = R⁸RⁱSiO_{2/2}
   T = R⁹SiO_{3/2};
   Tⁱ = RⁱSiO_{3/2};
   Q = SiO_{4/2};
   where R¹, R², R³, R⁴, R⁵, R⁶, R⁷, R⁸, and R⁹ are each independently selected from the group of monovalent hydrocarbon radicals having from 1 to 60 carbon atoms,
   Rⁱ is a monovalent hydrocarbon radical having from 2 to 60 carbon atoms and containing one or more acid functionalities selected from the group consisting of carboxylic acid-, phosphonic acid- and sulfonic acid-containing groups, their salts and combinations thereof, and subscripts a, b, c, d, e, f, and g are independently 0 or a positive number subject to the limitations b + d + f≥ 1 and a + b + c + d + e + f < 1000;
(b) 0.01 wt% to 10 wt% of a fragrance; and,
(c) an emulsion, suspension- or solution-forming amount of water and/or organic solvent.

There is also provided herein in another embodiment a process of making an aqueous emulsion, suspension or organic solvent solution comprising combining components (a)-(c), as described above, under emulsion-, suspension- or organic solvent solution forming conditions. The resulting emulsion, suspension or organic solvent solution can then be employed in a personal care, fabric care or home care product at any suitable level, e.g., those conventionally used in these categories of products. In particular embodiments, the emulsion or suspension can be an oil-in-water emulsion, e.g., an oil-in-water microemulsion.

In one embodiment herein, components (a)-(c) of the extended longevity fragrance composition are added separately to an aqueous gel suspension system such as a rinse-off conditioner, fragrance component (b) being present in the gel system together with the acid-functional silicone (a).

### DETAILED DESCRIPTION OF THE INVENTION

The present invention relates to an aqueous or non-aqueous emulsion or suspension or organic solvent solution, preferably a microemulsion or organic solvent solution, containing a fragrance-fixative amount of acid-functional silicone (a) and fragrance (b), and suitable for incorporation in any product for which a pleasing scent is desired, e.g., a personal care product such as those intended for application to skin or hair, a fabric care product such as a laundry detergent or fabric softener, or a home care product such as a hard surface cleaner. Such products upon drying will leave a film or residue upon the substrates to which they have been applied, the film or residue containing silicone compound (a) which then functions to retain, extend or prolong the scent of associated fragrance component (b).

The term "emulsion" as used herein designates a fine dispersion of minute droplets of one liquid in another in which it is not soluble or miscible.

The term "suspension" as used herein designates a mixture in which insoluble particles or droplets of one substance are present in another and wherein the particles or droplets do not readily or quickly settle or coalesce.

The expression "organic solvent solution" as used herein designates a homogeneous mixture of solute in organic solvent or solution of water and water miscible organic solvent.

The term "fixative" as used herein designates a substance that enhances, extends or prolongs the retention or longevity of a fragrance.

As used herein the expressions "fragrance retention" and "fragrance longevity" shall be understood to refer to the fixative property or substance in association with a fragrance, perfume or scent, expressed in levels of perceived fragrance intensity or effect after specific time intervals, as measured by a panel made up of experienced fragrance evaluators.

The expression "neutralized acid-functional silicone" as used herein shall be understood to designate the silicone of general formula (I) which, depending on context, applies to the silicone in its non-crosslinked form i.e., the form in which the silicone is present in the emulsion, suspension or organic solvent solution constituting the fragrance delivery composition, and to the silicone in its ionically cross-linked form, i.e., the form in which the silicone is present in the substantially dried (water-free) fragrance-containing composition or dried residue of such composition.

### A. Acid-Functional Silicone

In one embodiment herein, the acid-functional silicone of general formula (I) is such that each of R¹, R², R³, R⁴, R⁵, R⁶, R⁷, R⁸, and R⁹ are each independently selected from the group of monovalent hydrocarbon radicals having from 1 to 60 carbon atoms, more specifically 1 to 30 carbon atoms, even more specifically 1 to 12 carbon atoms and most specifically from 1 to 6 carbon atoms. In another embodiment, Rⁱ is a monovalent hydrocarbon radical containing from 2 to 60 carbon atoms, more specifically 2 to 30 carbon atoms, even more specifically from 2 to 16 carbon atoms and most specifically from 2 to 8 carbon atoms, and the acid functionalities are selected from carboxylic acid, phosphonic acid and sulfonic acid functionalities such as the non-limiting examples of ethylbenzylsufonate, decylcarboxylate and ethylphosphate. The subscripts a, b, c, d, e, f, and g are independently zero or a positive number subject to the limitations b + d + f ≥ 1, more specifically b+d+f≥ 2, and even more specifically b+d+f≥3, and a + b + c + d + e + f < 1000, more specifically, a + b + c + d + e + f < 500, even more specifically, a + b + c + d + e + f < 100, still more specifically a + b + c + d + e + f <50, and most specifically a + b + c + d + e + f < 30.

There is also provided herein an aqueous or non-aqueous emulsion, an aqueous or non-aqueous suspension, solution or organic solvent solution comprising acid functional silicone of general formula (I) wherein the subscripts a, d, e, f and g are 0 and the subscript b is 2 and subscript c is from 0 to 300, preferably from 1 to 300, more preferably from 1 to 100, and each Rⁱ is independently of the general formula (II): 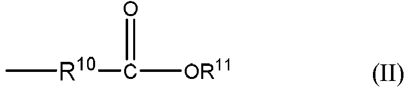 wherein
R¹⁰ is a divalent hydrocarbon moiety selected from alkyl, aryl, or alkylaryl containing up to about 35 carbon atoms, preferably up to about 20 carbon atoms and most preferably up to about 15 carbon atoms, wherein said ranges can optionally in one embodiment have lower endpoints of any one of 1, 3, 5, 6, 10 or 12, and R¹¹ is selected from hydrogen, alkali metal, an aminium group or a quaternized nitrogen group.

In another embodiment herein, the silicone of general formula (I) can have the more specific general formula (III): 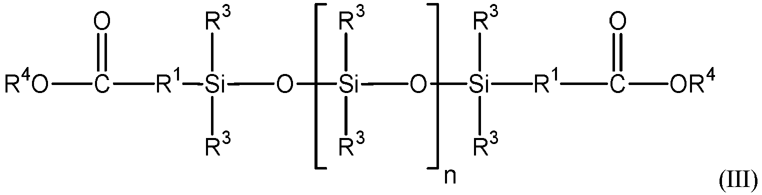 where the subscript n is from about 0 to about 300 and more specifically from 1 to 100, each R³ independently is a monovalent hydrocarbon radical containing from 1 to about 6 carbon atoms, each R¹ independently is a divalent moiety selected from alkyl, aryl or alkylaryl containing up to about 30 carbon atoms, more specifically up to about 20 carbon atoms and most specifically up to about 16 carbon atoms, wherein said ranges can in one separate embodiment have lower endpoints of any one of 1, 3, 5, 6, 10 or 12 carbon atoms, and each R⁴ independently is selected from hydrogen, alkali metal, calcium, magnesium, aminium group or quaternized nitrogen group.

In the longevity-enhancing extended fragrance delivery composition of the invention, the amount of acid-functional silicone (a) of general formula (I) sufficient to extend the longevity of fragrance (b) can vary widely depending upon the fixative properties of the particular acid-functional silicone (a) selected, the nature of its associated fragrance (b) and if present, other components of the emulsion or suspension and/or components of the product in which the composition is incorporated as will be understood by those skilled in the art. The fragrance longevity-enhancing amounts are from 0.01 wt% to 60 wt%. More specific embodiments comprise from about 0.01 wt% to about 40 wt%, and most specifically from about 0.02 wt% to about 50 wt%, said weight percents being based on the total weight of the aqueous emulsion, suspension or organic solvent solution.

In one embodiment herein, the silicone of general formula (III) described above has a degree of polymerization as defined by subscript n, e.g., as measured by NMR, GPC or gel permeation chromatography, of from about 2 to about 300, more specifically from about 10 to about 250 and most specifically from about 40 to about 150.

In one embodiment, the aqueous emulsion, suspension or organic solvent solution of acid-functional silicone (a) and fragrance (b) has a pH of from about 6 to about 12, more specifically from about 6.5 to about 10 and in one particular embodiment, from about 5 to about 9. When it is desired that the longevity-enhancing fragrance delivery composition herein form a continuous film, all or some of the acid functional groups can be neutralized with a base. The neutralized acid functional groups through ionic forces will aggregate to form a continuous film. All or some of the acid functional groups can be neutralized, e.g., greater than 30, more specifically greater than 50 and still more specifically greater than 80 up to 100 mole percent of such groups. The acid functional groups can be neutralized with a base such as the non-limiting examples of NaOH, KOH, triethanol amine, clay particles such as betntonite and laponite, silica particles (due to NaOH), boron nitride particles (due to ammonia generation), metal oxides such as Al₂O₃, Fe₂O₃, Fe₃O₄, TiO₂, ZnO and Mgo, inorganic pigments such as cobalt blue and admium orange, and organic particles such as polyacrylate, and the like, to provide neutralized acid-functional silicone (a). If the pH of the aqueous emulsion, suspension or organic solvent solution or that of the product containing the aqueous emulsion, suspension or organic solvent solution is basic, an appropriate acid, such as the non-limiting example of acetic acid, can be used to decrease the pH to a suitable level.

The aqueous emulsion, suspension or organic solvent solution can contain acid-functional silicone (a) of general formula (I) in an amount of from 0.01wt% to about 50 wt%, more specifically from about 1 wt% to about 30 wt% and most specifically from about 10 wt% to about 25 wt% based on the total weight of the aqueous emulsion or suspension.

### B. Fragrance

The at least one fragrance compound (b) can be obtained from a source selected from the group consisting of essential oils, flower oils, natural extracts from resins, gums, balsams, beans, mosses, plants, ambergris, musk, synthetic aromatic materials and combinations thereof. It is understood herein that the fragrance (b) can comprise any one or more of the entire essential oil, flower oil, or natural extract, or can comprises one or more components thereof. Typically, fragrance materials are supplied as concentrates, which generally contain up to about 3 percent fragrance by weight. In addition, the fragrance (b) can comprise the synthetic version of any one or more of the compounds of any of the essential oils, flower oils, or natural extracts described herein. While the specific fragrance scent of the at least one fragrance compound can be any scent desired by the user, or any scent desirable for personal care products and/or fabric treatment, it is preferable that the scent be selected from the group consisting of masking, citrus, floral, spicy, lavender, woody, mossy, oriental, herbal, leather-tobacco and aldehydic notes and combinations thereof. The at least one fragrance compound (b) is present in an amount of from 0.01wt % to 10 wt %, more specifically from about 0.05wt % to about 2 wt % and most specifically from about 0. 1wt % to about 1 wt %, based on the total weight of the emulsion, suspension or organic solvent solution.

The aqueous emulsion or suspension of the present invention comprises fragrance (b) materials to improve the fragrance longevity of the aqueous emulsion or suspension, and to provide extended fragrance longevity to products in which the aqueous emulsion, suspension is incorporated.

The concentration of the fragrance (b) is preferably sufficient to provide olfactory detection of the fragrance (b) upon release of the perfume from the composition. An individual fragrance (b) or mixtures of these materials may be used herein.

The fragrance (b) materials for use in the aqueous emulsion or suspension of the present invention include any known perfumes in the art or any otherwise effective perfume materials. Many of the perfumes described herein, along with their odor characters and their physical and chemical properties such as boiling point and molecular weights, are disclosed in "Perfume and Flavor Chemicals (Aroma Chemicals)," S. Arctander, published by the author, 1969.

Nonlimiting examples of suitable fragrance (b) for use herein include anethole, benzaldehyde, decyl aldehyde, amyl acetate, benzyl acetate, benzyl alcohol, benzyl formate, benzyl propionate, iso-bornyl acetate, camphene, cis-citral (neral), citronellal, citronellol, citronellyl acetate, paracymene, decanal, dihydrolinalool, dihydromyrcenol, dimethyl phenyl carbinol, eucalyptol, geranial, geraniol, geranyl acetate, geranyl nitrile, helional, cis-3-hexenol, cis-3-hexenyl acetate, dipropylene glycol, diethyl phthalate, phenyl ethyl acetate, dihydrocitronellal, d-limonene, linalool, linalool oxide, tetra-hydro linalool, linalyl acetate, linalyl propionate, methyl anthranilate, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, prenyl acetate, manjantol, ambrettolide, phenyl ethyl alcohol, phenyl acetaldehyde, alpha-pinene, beta-pinene, gamma-terpinene, alpha-terpineol, beta-terpineol, terpinyl acetate, vertenex (para-tertiary-butyl cyclohexyl acetate), alpha damascone, damascone beta, undecalactone, undecylenic aldehyde, amyl cinnamic aldehyde, isoamyl salicylate, beta-caryophyllene, cedrene, cinnamic alcohol, cymal, dimethyl benzyl carbinyl acetate, dimethyl benzyl carbinol, ethyl vanillin, eugenol, iso-eugenol, dihydro-norcyclopentadienyl acetate, dihydro-nor-cyclopentadienyl propionate, heliotropine, cyclohexyl salicylate, 3-cis-hexenyl salicylate, hexyl salicylate, lilial (para-tertiarybutyl-alpha-methyl hydrocinnamic aldehyde), gamma-methyl ionone, nerolidol, patchouli alcohol, phenyl hexanol, beta-selinene, trichloromethyl phenyl carbinyl acetate, triethyl citrate, vanillin, veratraldehyde, 2-methyl-3-(para tert butylphenyl)-propionaldehyde, benzophenone, benzyl salicylate, ethylene brassylate, galaxolide (1,3,4,6,7,8-Hexahydro-4,6,6,7,8,8-hexamethyl-cyclopenta-gamma-2-benzopyra n), hexyl cinnamic aldehyde, lyral (4-(4-hydroxy-4-methylpentyl)-3-cyclohexene- 10-carboxaldehyde), methyl cedrylone, dihydro isojasmonate, methyl dihydro jasmonate, methyl-beta-naphthyl ketone, gamma decalactone, musk indanone, musk ketone, musk tibetene, phenylethyl phenyl acetate, cyfleural S6MC, PE isobutyrate, PE propionate, tripal, LRG 201, ligustral, and mixtures thereof.

Other suitable fragrances for use herein include any known natural oil fragrance material known in the art. Nonlimiting examples of natural oils include terpenes; patchouli oils; french crop oils; citrus oils; clove derivatives; gums, mosses, and resins; and general natural oils. Specific examples of terpenes include, but are not limited to, orange terpenes, lemon terpenes, clementine terpenes, cedarwood terpenes, grapefruit terpenes, grapefruit terpenes distilled, and mixtures thereof. Specific examples of patchouli oils include, but are not limited to, patchouli decolorized, patchouli oil md, patchouli enhancer PG 67002, patchouli B50010 coeur (798304), patchouli booster AN113418, patchouli oil I.F.indo A, and mixtures thereof. Specific examples of french crop oils include, but are not limited to, lavandin, lavandin grosso, spike lavender, clary sage, and mixtures thereof. Specific examples of citrus oils include, but are not limited to, Italian orange phase oil, cold pressed orange oil, orange oil tarocco 5X (10982), lemon oil, lemon c.p., tangerine distilled oil, tangerine oil, and mixtures thereof. Specific examples of clove derivatives include, but are not limited to, eugenol, iso eugenol, iso eugenol acetate, clove stem oil, clove bud oil, and mixtures thereof. Specific examples of gums, mosses, and resins include, but are not limited to, styrax resin 50% in DPG, olibanium resinoid 80%, peru balsam, labdanum clair, styrax white, oakmoss 25%, geranium S, and mixtures thereof. Specific examples of general natural oils include, but are not limited to, citronella, petitgrain, elemi oil, galbanum 50%, ylang ylang, rosemary, menthol, menthol natural, caraway, cananga, nutmeg, coriander, cassia oil, celery seed oil, and mixtures thereof.

### B. Fragrance Delivery Composition in the Form of an Emulsion or Suspension

When preparing an aqueous emulsion or suspension of the fragrance delivery composition, the water component thereof can comprise the balance of the composition when the amounts of components (a) and (b) are chosen. More specifically, the aqueous emulsion- or suspension forming amount of water can range from about 25 wt% to about 99.9 wt%, more specifically from about 40 wt% to about 99 wt% and most specifically of from about 50 wt% to about 99 wt% of the total fragrance delivery composition. Similarly, in the case of an organic solvent emulsion or suspension, the organic solvent can be present in the fragrance delivery composition within the aforestated ranges.

The emulsion or suspension can further comprise an optional emulsifier or suspending agent (d), e.g., a surfactant selected from the group consisting of non-ionic surfactant, ionic surfactant, anionic surfactant, cationic surfactant, amphoteric surfactant, zwitterionic surfacant and combinations thereof.

Suitable surfactants (d) may be exemplified by anionic surface active agents such as hexylbenzenesulfonic acid, octylbenzenesulfonic acid, decylbenzylsulfonic acid, dodecylbenzenesulfonic acid, cetylbenzenesulfonic acid, myristylbenzenesulfonic acid and their salts; cationic surface active agents such as octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow trimethylammonium hydroxide, coconut oil trimethylammonium hydroxide; nonionic surface active agents such as those of the polyester series, as well as ethylene oxide adducts of diethylene glycol trimethyl nonanol, polypropylene glycol, polyethylene glycol, polyoxyalkylene sorbitan ester, polyoxyalkylene alkyl ester, polyoxyalkylene alkyl phenol, and polyoxyalkylene alkyl ether. Such surfactants can be used singly or as a mixture of two or more agents. The use of nonionic surface active agents for emulsification is particularly preferable when using the present composition as an additive for personal care products. There are no limitations concerning the amount of emulsifier or suspending agent (d), but more specifically it is in the range of 0.01 to 50 parts by weight and even more preferably in the range of 0.1 to 25 parts by weight per 100 parts by weight of the aqueous emulsion or suspension. In one embodiment of emulsion or suspension, acid-functional silicone (a) can be up to twice the amount of emulsifier/suspending agent (d) by weight. When the weight ratio of acid-functional silicone (a) to emulsifier/suspending agent (d) is greater than double, the stability of the emulsion or suspension may decrease.

In one non-limiting embodiment herein, the aqueous or organic solvent emulsion or suspension can be dried so as to contain little to no water and/or organic solvent, e.g., less than 1 wt% thereof.

In one non-limiting embodiment herein the aqueous or organic solvent emulsion or suspension can be a clear and/or transparent emulsion/suspension.

In order to impart to the emulsions or suspensions of the present invention some additional desired characteristic(s), one or more optional components that have physical and chemical compatibility may be added. Some specific examples of components that may be optionally added thereto are preservatives, viscosity modifiers, viscosity donating agents, antioxidant agents, stylization agents, pearlizing agents, opacifying agents, dyestuffs, vitamins, active agents, cleansing agents, conditioning agents, pH-adjusting agents and the like.

In yet one more specific embodiment, the emulsion or suspension can further optionally comprise a filler. The filler can be of the reinforcing or non-reinforcing type or combination thereof. In one embodiment, the filler is selected from the group consisting of fumed silica, precipitated silica, clay, carbon black, silicone resins, calcium carbonates and combinations thereof.

Any of the conventional or otherwise known procedures for preparing an aqueous or organic solvent emulsion or suspension can be utilized herein. In one embodiment, the emulsion or suspension may be prepared by combining a fragrance longevity-enhancing amount of acid-functional silicone (a), fragrance (b) and water and/or organic solvent (c) under emulsion- or suspension-forming conditions, e.g., employing optional emulsifier or suspending agent (d).

In one non-limiting embodiment herein, there is provided an oil-in-water emulsion or suspension in which emulsifier or suspending agent (d) forms a monolayer at the interface between the oil phase (acid functional silicone and fragrance) and the water phase with the hydrophobic tails of molecules (d) dissolved in the oil phase and their hydrophilic heads dissolved in the aqueous phase. In one embodiment, the dispersed oil phase of the aqueous emulsion or suspension has a diameter of from 1nm to about 500 nm, more specifically from about 5nm to about 300 nm, and still more specifically from about 10 nm to about 250 nm.

The combining of components (a), (b) and (c) and optional component (d) under emulsion or suspension-forming conditions can comprise simple mixing the components (a)-(d) to form the aqueous emulsion or suspension, or the use of mechanical energy, e.g., vigorous automated mixing or agitation to form the aqueous emulsion or suspension. Such automated mixing or agitation can be used to emulsify or suspend the aqueous emulsion or suspension components (a)-(c) under low to high shear and may be adjusted for the desired viscosity and sensory feel of the emulsion or suspension. This may be achieved, for example, by subjecting the components (a)-(c) to a moderate to high shearing force. High shear may be applied using, for example, a Sonolator apparatus, a Gaulin Homogenizer or a Micro Fluidizer apparatus. Optionally, one or more carrier solvent may be added to the aqueous emulsion or suspension prior to the shearing. Some non-limiting examples of such carrier solvent are those selected from the group consisting of hydroxyl containing organic compounds comprising alcohols, glycols, polyhydric alcohols and polymeric glycols and mixtures thereof that are liquid at room temperature, e.g. about 25°C., and about one atmosphere pressure. Preferably the solvent is selected from the group consisting of ethylene glycol, ethanol, propyl alcohol, iso-propyl alcohol, propylene glycol, dipropylene glycol, tripropylene glycol, butylene glycol, iso-butylene glycol, methyl propane diol, glycerin, sorbitol, polyethylene glycol, polypropylene glycol mono alkyl ethers, polyoxyalkylene copolymers and mixtures thereof.

However, usually, the preparation of the emulsion or suspension herein does not require mechanical energy and can be formed simply by mixing components (a)-(c). Two different methods may optionally be used for preparing the emulsion or suspension of the present invention, namely, homogenization under high pressure which typically results in transparent products, and the Phase Inversion Temperature (PIT) method which provides transparent products having low contents of component (d). These two methods reduce the concentration of surfactant (d) necessary for obtaining the aqueous emulsion described herein.

In one specific embodiment, the procedure for making the aqueous emulsion or suspension containing fragrance (b) can comprise combining components (a)-(c) simultaneously. The step of combining these components can be conducted relatively quickly, e.g., for a few seconds such as 3 or 4 seconds, up to 5 minutes. The step of combining can be conducted at ambient temperature (~20°C) up to about 100°C, more specifically from 20°C to about 80°C and still more specifically from about 25°C to about 60°C. In another embodiment of the process of making the emulsion or suspension, components (a)-(c) or (a)-(d) may be combined in any manner, e.g., simultaneously or separately. In another embodiment, components (a) and (c) and optionally emulsifier or suspension agent (d) and fragrance (b) may be provided in a gel system, e.g., a hydrogel system such that components (a) and (b) are suspended therein.

Non-limiting examples of hydrogen include agarose or a water-soluble low-substituted cellulose ether which may include methyl cellulose and related thickeners; crosslinked acrylic acid homopolymers; crosslinked copolymers of (meth)acrylic acid and of (C1-C6)alkyl acrylate; nonionic homopolymers and copolymers containing ethylenically unsaturated monomers of ester and/or amide type; ammonium acrylate homopolymers or copolymers of ammonium acrylate and of acrylam ide; (meth)acrylamido(C1-C4)alkylsulphonic acid homopolymers and copolymers; crosslinked methacryloyl(C1-C4)alkyltri(C1-C4)alkylammonium homopolymers and copolymers. Particlulate thickeners may also be used. Also, naturally derived polymers and polymers produced by fermentations may be used such as polysaccharide gums, xanthan gum, pullulan gum, sclerotium gum, carrageenan gum, locust bean gum, alginate, gellan gum, cellulose, carboxymethylcellulose. hydroxyethylcellulose, pectins, starch, chitosan, gelatin, and their combinations.

In one non-limiting embodiment herein, the process step of combining components (a)-(c) and optionally component (d) to make the aqueous emulsion or suspension of the present invention can comprise heating the surfactant (d) and acid-functional silicone (a) to 50 °C and mixing, adding 1 wt% NaCl water solution in four parts under stirring. After that, adding 2.5 wt% NaOH water solution under stirring to get the neutralized aqueous emulsion or suspension. After the mixing, one should cool the composition to room temperature and use an acid to adjust pH. Fragrance (b) can thereafter be introduced into the aqueous emulsion or suspension under stirring to form the aqueous emulsion or suspension.

### C. Fragrance Delivery Composition in the Form of an Organic Solvent Solution

In addition to being formulated as aqeous or organic solvent emulsions or suspensions, the fragrance delivery compositions can be provided as organic solvent solutions of acid-functional silicone (a) and fragrance (b).

Organic solvents include water soluble solvents and water insoluble solvents. Water soluble solvents include, but are not limited to, ethanol, 1-propanol, isopropanol, butyl alcohol, triethanolamine, acetic acid, ethylamine, ethylene glycol, propylene glycol, dipropylene glycol, glycerine, methanol, acetaldehyde, acetone, acetonitrile, 1,2-butanediol, 1,3-butanediol, 1,4-butanediol, 2-butoxyethanol, butyric acid, diethanolamine, diethylenetriamine, dimethylformamide, dimethoxyethane, dimethyl sulfoxide, 1,4-dioxane, formic acid, furfuryl alcohol, methyl diethanolamine, methyl isocyanide, 1,3-propanediol, 1,5-pentanediol, propanoic acid, pyridien, tetrahydrofuran, diethylene glycol, triethylene glycol, ethylhexylglycerine and caprylyl glycol.

Water insoluble solvents include, but are not limited to, dimethicone, dimethiconol cylcodimethicone, decamethylcyclopentasiloxane, 1,1,1,3,5,5,5-heptamethyl-3-ethyltrisiloxane, 1,1,1,3,5,5,5-heptamethyl-3-octyltrisiloxane methyltris(trimethlylsiloxy)silane, isododecane, isopropylmyristate, diisoproypyl adipate, dicaprylyl carbonate, pentane, cyclohexane, hexane, heptane, isohexadecane, ethyl acetate, mineral spirit, petroleum ether, carbon tetracholoride, chloroform, chlorobenzene, diethyl ether, methyl t-butyl ether (MTBE), methylene chloride, nitromethane, toluene, o-xylene, m-xylene and p-xylene.

Acid-functional silicone (a) can represent from 0.01 to 50 wt%, more specifically of from 0.1 to 20 wt% and still more specifically from 1 to 10 wt% of the total weight of fragrance delivery composition; fragrance (b) can represent from 0.01 to 50 wt%, more specifically from 0.1 to 30 wt% and still more specifically from 10 to 20 wt% of the total weight of fragrance delivery composition; and, organic solvent (c) or mixture of water and organic solvent (c) can represent from about 10 to 99.98 wt%, more specifically from 50 to 99.8 wt% and still more specifically from 70 to 90 wt% of the total weight of fragrance delivery composition.

The order of adding acid-functional silicone (a), fragrance (b) and organic solvent (c) or mixture of water and organic solvent (c) in most cases will not affect system homogeneity or the fragrance retention effect.

### D. Personal Care Products

In one embodiment herein, the fragrance delivery composition in the form of an aqueous emulsion, aqueous suspension or organic solvent solution can be added to a personal care formulation for use in a personal care application for skin and/or hair. Some such non-limiting examples of personal care applications are selected from the group consisting of perfumes, deodorants, antiperspirants, antiperspirant/deodorants, shaving products, skin lotions, moisturizers, toners, bath products, cleansing products, shampoos, conditioners, mousses, styling gels, hair sprays, hair dyes, hair color products, hair bleaches, waving products, hair straighteners, nail polish, nail polish remover, nail creams and lotions, cuticle softeners, sunscreen, insect repellent and anti-aging products, lipsticks, foundations, face powders, eye liners, eye shadows, blushes, makeup, pet grooming products, and mascaras.

In a more specific embodiment, the aqueous emulsion, aqueous suspension or organic solvent solution can be added to a personal care formulation such as the non-limiting examples of personal care formulations used in personal care applications selected from the group consisting of perfume, shampoo, conditioner, hair serum, skin cream, skin cleansing formulations, and skin serum.

In one non-limiting embodiment, the components of the aqueous emulsion, aqueous suspension or organic solvent solution can in part be present in the personal care formulation and then followed by the addition of the remaining components of the aqueous emulsion, aqueous suspension or organic solvent solution, and then followed by mixing and/or application of mechanical energy, as described herein, to/of the personal care formulation containing the components of the aqueous emulsion, aqueous suspension or organic solvent solution to emulsify or dissolve, as the case may be, the components (a)-(c) and form the aqueous emulsion, aqueous suspension or organic solvent solution in the personal care formulation.

In yet another non-limiting embodiment, all of the components of the aqueous emulsion, aqueous suspension or organic solvent solution can in part be present in the personal care formulation and then followed by mixing and/or application of mechanical energy, as described herein, to/of the personal care formulation containing the components of the emulsion, aqueous suspension or organic solvent solution to emulsify the components (a)-(d) and form the aqueous emulsion, aqueous suspension or organic solvent solution in the personal care formulation.

The amount of fragrance delivery composition that can be added to a personal care formulation can vary widely depending on the specific formulations and the desired properties of its resultant personal care application, but in one non-limiting embodiment, the amount of such composition that can be added to a personal care formulation can be of from about 0.01 wt % to about 100 wt %, more specifically from about 0.1 wt% to about 100 wt% and most specifically from about 0.2 wt% to about 100 wt%, based on the total weight of the personal care formulation.

One non-limiting example of a component of a personal care formulation herein can be a suitable emollient compound, which can include any fluid that provides emollient properties, that is, that when applied to skin, tends to remain on the surface of the skin or in the stratum corneum layer of the skin to act as lubricants, reduce flaking and to improve the appearance of the skin. Emollient compounds are generically known and include, for example, hydrocarbons, such as for example, isododecane, isohexadecane and hydrogenated polyisobutene, organic waxes, such as for example, jojoba, silicone fluids, such as, for example, cyclopentasiloxane, dimethicone and bis-phenylpropyl dimethicone, esters, such as, for example, octyldodecyl neopentanoate and oleyl oleate, as well as fatty acids and alcohols, such as for example, oleyl alcohol and isomyristyl alcohol. Some non-limiting examples of hydrophilic emollients are those selected from the group consisting of glycerine, sorbitol, aqueous solution of moisturizing additives and combinations thereof.

Some further non-limiting examples of components of a personal care formulation herein can be skin conditioners, moisturizers and surfactants. Illustrative conditioners include mineral oil, petrolatum, vegetable oils (such as soybean or maleated soybean oil), dimethicone, dimethicone copolyol, cationic monomers and polymers (such as distearyl dimethyl ammonium chloride). Illustrative moisturizers are polyols such as sorbitol, glycerin, propylene glycol, ethylene glycol, polyethylene glycol, polypropylene glycol, 1,3-butane diol, hexylene glycol, isoprene glycol, xylitol, fructose and mixtures thereof.

Further additives for the personal care formulations herein may be selected from retinoids (e.g. retinol and retinyl linoleate), ascorbic acid and derivatives thereof, herbal extracts, pigments, vitamins, such as, for example, Vitamin A, Vitamin C and Vitamin E, sunscreen or sunblock compounds, such as, for example, titanium dioxide, zinc oxide, oxybenzone, octylmethoxy cinnamate, butylmethoxy dibenzoylm ethane, p-aminobenzoic acid and octyl dimethyl-p-aminobenzoic acid and combinations thereof.

In another embodiment, the personal care formulation can comprise thickeners such as the non-limiting example of hydrophobically modified cellulose, Methocel from Dow; Xanthan Gum can be used to thicken the system and kept the system clear without losing fragrance retention and other hair care benefits. The thickened system can be used as hair serum.

In a preferred embodiment of the present invention, cosmetic components known in the art may be incorporated in the personal care formulation for improving skin appearance. These components can be any of anti-blotching, anti-aging, eye contour, slimming, soothing/sunburn, anti-irritating, skin firming and lifting, free radical scavengers, hydratives, vitamins and anti-oxidants and minerals. Amounts of any of these aforementioned personal care formulation materials may range anywhere from 0.0001 to 5% by weight of the personal care formulation.

In one embodiment herein, the personal care formulation containing the fragrance delivery composition itself or a personal care application containing the personal care formulation can be applied to a skin or hair surface and then dried so as to form an ionically crosslinked film on the skin or hair surface. As the water or organic solvent is removed during evaporation, the salt endgroups on the silicone aggregate. The ionic domains become less mobile and each domain becomes tethered to each other through the polydimethylsiloxane backbones. The resulting solid film is resistant to removal and solvation by water and organic solvents.

The ionically crosslinked film on the skin, hair, fabric or hard surface can retain the fragrance for a prolonged period of time, such as the non-limiting period of from 6 hours to 100 hours, more specifically from 10 hours to 80 hours and most specifically from 24 hours to 72 hours.

### E. Home Care Products

In one embodiment herein, there is provided a home care formulation comprising the fragrance delivery composition described herein. There is also provided a hard surface comprising the fragrance delivery composition described herein. The description and properties of the fragrance delivery composition as used in personal care and fabric treatment applications apply equally to home care applications. Some non-limiting examples of home care applications include those selected from the group consisting of laundry detergent and fabric softener, dishwashing liquids, wood and furniture polish, floor polish, tub and tile cleaners, toilet bowl cleaners, hard surface cleaners, window cleaners, antifog agents, drain cleaners, auto-dishwashing detergents and sheeting agents, carpet cleaners, prewash spotters, rust cleaners, automotive care products, leather cleaners, leather conditioners, room air fresheners, odor maskers and scale removers.

In another embodiment, there is provided herein a process comprising treating a hard surface with an aqueous emulsion, aqueous suspension or organic solvent solution, such as the fragrance delivery composition described herein, and the fragrance delivery composition made by the processes described herein. The method of treating can comprise applying the fragrance delivery composition to the hard surface and drying the composition so as to form an ionically crosslinked film on the surface. The ionically crosslinked film can be the same as that described above, and can have the same ranges of fragrance retention properties as that described above.

Methods of applying the fragrance delivery composition are effected by any industrially acceptable manner, preferably by spreading, foaming and/or spraying means.

The hard surface can be selected from the non-limiting group consisting of sythenthic polymeric surfaces, natural stone, wood, tile, and porcelain.

### F. Fabric Treatment Products

In another embodiment, there is provided herein a process comprising treating a fabric with an aqueous emulsion, aqueous suspension or organic solvent solution, such as described herein, and the fragrance delivery composition made by the processes described herein. The method of treating can comprise applying the fragrance delivery composition to the fabric and drying the composition so as to form an ionically crosslinked film on the fabric. The ionically crosslinked film can be the same as that described above, and can have the same ranges of fragrance retention properties as that described above.

Methods of applying the fragrance delivery composition are effected by any industrially acceptable manner, preferably by spreading, padding, foaming and/or spraying means, and can also comprise applying fragrance delivery composition to one side of the fabric such as front-coating or back-coating, or immersion of the fabric via dipping.

Padding is defined as a process in which the fabric is first passed through a padder containing the fragrance delivery composition wherein the composition is applied, and the fabric is then squeezed between heavy rollers to remove any excess of the aqueous emulsion or suspension.

The process of applying the fragrance delivery composition described herein can optionally be affected, for example, either during the dying or the finishing stages of the textile substrate manufacture.

The fabric can be selected from the non-limiting group consisting of synthetic textiles, natural textiles and blends thereof. Non-limiting examples of textile substrates that can be beneficially used in the context of the present invention include wool, silk, cotton, linen, hemp, ramie, jute, acetate fabric, acrylic fabric, latex, nylon, polyester, rayon, viscose, spandex, metallic composite, carbon or carbonized composite, and any combination thereof. Preferable non-limiting examples of textile fabrics which were shown to be suitable for use in the context of the present invention include, without limitation, cotton, polyester, and combinations thereof.

### EXAMPLES

### Preparation of TMS protected 10-undecylenic acid (UATMS)

To a 500 mL round bottom flask 200 g of undecylenic acid was charged. The flask was heated to 80 °C and 109.5 g of hexamethyldisilazane (HMDZ) was added dropwise via an addition funnel Once all the HMDZ was added the reaction temperature was increased to 90 °C and stirred under nitrogen for 4 hrs, cooled to room temperature and stirred overnight. The reaction was determined to be complete by the dissappearence of the UA peak in the GC chromatogram. The material was stripped at 135 °C at 50 torr with a nitrogen sparge through a short path distillation head. A clear brown low viscosity fluid was obtained.

### Preparation of TMS protected 3-butenoic acid (BATMS)

3-butenoic acid (200 g) was added to a 500 mL round bottom flask and agitated with an overhead stirrer. The reaction of kept under a nitrogen blanket. Hexamethyldisilazane (HMDZ) (117.2 g) was added dropwise at 80 °C. After the addition was complete the reaction was heated to 90 °C and stirred for 4 hrs, then cooled to room temperature and held overnight. The reaction was determined to be complete by GC. The product was then distilled through a 5 plate Oldershaw column to obtain pure 3-butenoictrimethylsilylester as a clear colorless fluid with a boiling point of 60 °C at 27 torr.

### Synthetic Example 1: Preparation of D100

A hydride functional siloxane (289.38 g) with the structure H(CH₃)₂SiO[Si(CH₃)₂)O]₁₀₀Si(CH₃)₂H, and 22.27 g of UATMS were combined into a 500 mL round bottom flask. The flask was heated to 85 °C and Karstedt's catalyst (10 ppm based on Pt) was added. The reactor was kept under a nitrogen blanket and allowed to react for 18 hrs. The reaction was determined to be completed by digesting a 1 mL sample in a caustic digestion tube. When no H₂ evolution was observed all Si-H groups were deemed to be reacted. NMR also indicated complete reaction by the disappearance of the Si-H peak at 4.7 ppm. 39.94 g of ethanol was added and the reactor was heated to 60 °C for 18 hrs. The material was then stripped with a nitrogen sparge at 50 torr and 135 °C for 2 hrs to remove the ethoxytrimethylsilane. The resulting fluid was light brown in color with a slight haze and a viscosity of 372 cP.

### Synthetic Example 2: Preparation of MD15D^{∗}5.5M

TMS protected 10-undecylenic acid (UATMS) (153.09 g) was added in a 500 mL 4-neck flask equipped with a N₂ blanket, condenser and a thermocouple. A hydride functional silicone with the structure (CH₃)₃SiO[Si(CH₃)₂)O]₁₅[Si(CH₃)(H)O]_{5.5}Si(CH₃)₃ (146.98 g) was added slowly using an addition funnel. The temperature controller was set to 85 °C. The reaction was catalyzed with Karstedt's catalyst (10 ppm of Pt). After 18 hr the reaction was completed as indicated using a caustic fermentation tube. The reaction then cooled to 60 °C and 100 g of ethanol was added and stirred at for 4 hrs. The product was stripped for 2 hrs at 80 °C using a short path distillation head, nitrogen sparge and an overhead stirrer at 50 torr. The resulting material was a clear light brown fluid.

### Synthetic Example 3: Preparation of D25

TMS protected 10-undecylenic acid (UATMS) (67.1 g) was added in a 250 mL 4-neck flask equipped with a N₂ blanket, condenser and a thermocouple. A hydride functional silicone with the structure H(CH₃)₂SiO[Si(CH₃)₂)O]₅₀Si(CH₃)₂H (200 g) was added to the pot. The temperature controller was set to 85 °C. The reaction was catalyzed with Karstedt's catalyst (10 ppm of Pt). After 18 hrs the reaction was completed, indicated by using a caustic fermentation tube. The reaction then cooled to 60 °C and 18 ml of ethanol was added and stirred at for 4 hrs. The product was stripped for 4 hrs at 110 °C using a short path distillation head, nitrogen sparge and an overhead stirrer at 50 torr. The resulting product was a clear, light brown fluid.

### Synthetic Example 4: Preparation of D50

TMS protected 10-undecylenic acid (UATMS) (34.72 g) was added in a 250 mL 4-neck flask equipped with a N₂ blanket, condenser and a thermocouple. A hydride functional silicone with the structure H(CH₃)₂SiO[Si(CH₃)₂)O]₅₀Si(CH₃)₂H (200 g) was added to the pot. The temperature controller was set to 85 °C. The reaction was catalyzed with Karstedt's catalyst (10 ppm of Pt). After 18 hrs the reaction was completed, indicated by using a caustic fermentation tube. The reaction then cooled to 60 °C and 18 ml of ethanol was added and stirred at for 4 hrs. The product was stripped for 4 hrs at 110 °C using a short path distillation head, nitrogen sparge and an overhead stirrer at 50 torr. The resulting product was a clear, medium brown fluid.

### Synthetic Example 5: Preparation of D200

TMS protected 10-undecylenic acid (UATMS) (10.96 g) was added in a 500 mL 4-neck flask equipped with a N₂ blanket, condenser and a thermocouple. A hydride functional silicone with the structure H(CH₃)₂SiO[Si(CH₃)₂)O]₂₀₀Si(CH₃)₂H (285 g) was added to the pot. The temperature controller was set to 85 °C. The reaction was catalyzed with Karstedt's catalyst (10 ppm of Pt). After 18 hrs the reaction was completed, indicated by using a caustic fermentation tube. The reaction then cooled to 60 °C and 19.74 ml of ethanol was added and stirred at for 4 hrs. The product was stripped for 4 hrs at 110 °C using a short path distillation head, nitrogen sparge and an overhead stirrer at 50 torr. The resulting product was a clear, medium brown fluid.

### Synthetic Example 6: Preparation of D3

TMS protected 10-undecylenic acid (UATMS) (8.1 g) was added in a 500 mL 4-neck flask equipped with a N₂ blanket, condenser and a thermocouple. A hydride functional silicone with the structure H(CH₃)₂SiO[Si(CH₃)₂)O]₃₀₀Si(CH₃)₂H (292 g) was added to the pot. The temperature controller was set to 85 °C. The reaction was catalyzed with Karstedt's catalyst (10 ppm of Pt). After 18 hrs the reaction incomplete and an additional 0.44 g of UATMS and Karstedt's catalyst (2 ppm of Pt) was added. The reactor was stirred at 85 °C for an additional 4 hrs. The reaction was completed, indicated by using a caustic fermentation tube. The reaction then cooled to 60 °C and 14.5 ml of ethanol was added and stirred at for 4 hrs. The product was stripped for 4 hrs at 110 °C using a short path distillation head, nitrogen sparge and an overhead stirrer at 50 torr. The resulting product was a clear, dark brown fluid.

### Synthetic Example 7: Preparation of D500

TMS protected 10-undecylenic acid (UATMS) (4.59 g) was added in a 500 mL 4-neck flask equipped with a N2 blanket, condenser and a thermocouple. A hydride functional silicone with the structure H(CH3)2SiO[Si(CH3)2)0]500Si(CH3)2H (295 g) was added to the pot. The temperature controller was set to 85 oC. The reaction was catalyzed with Karstedt's catalyst (10 ppm of Pt). After 18 hrs the reaction was completed, indicated by using a caustic fermentation tube. The reaction then cooled to 60 oC and 8.27 ml of ethanol was added and stirred at for 4 hrs. The product was stripped for 4 hrs at 110 oC using a short path distillation head, nitrogen sparge and an overhead stirrer at 50 torr. The resulting product was a slightly hazey, medium brown fluid.

### Synthetic Example 8: Preparation of MD15D^{∗}5.5M

TMS protected butenoic acid (BATMS) (82.83 g) was added in a 250 mL 4-neck flask equipped with a N2 blanket, condenser and a thermocouple. A hydride functional silicone with the structure (CH3)3SiO[Si(CH3)2)O]15[Si(CH3)(H)O]5.5Si(CH3)3 (82.63 g) was added slowly using an addition funnel. The temperature controller was set to 85 oC. The reaction was catalyzed with Karstedt's catalyst (10 ppm of Pt). After 1.5 hr the reaction was recatalyzed with another 10 ppm Pt and stirred for 48hrs. The reaction was completed as indicated using a caustic fermentation tube. The reaction then cooled to 60 oC and 100 g of ethanol was added and stirred at for 4 hrs. The product was stripped for 2 hrs at 80 oC using a short path distillation head, nitrogen sparge and an overhead stirrer at 50 torr. Diatomaceous earth (1.7 g) was added and the solution was filtered through a 5 µM filter. The resulting material was a clear colorless fluid.

### Synthetic Example 9: Preparation of D25

TMS protected butenoic acid (BATMS) (63.52 g) was added in a 500 mL 4-neck flask equipped with a N2 blanket, condenser and a thermocouple. A hydride functional silicone with the structure H(CH3)2SiO[Si(CH3)2)O]25Si(CH3)2H (186.81 g) was added slowly using an addition funnel. The temperature controller was set to 85 oC. The reaction was catalyzed with Karstedt's catalyst (10 ppm of Pt). After 1.5 hr the reaction was completed as indicated using a caustic fermentation tube. The reaction then cooled to 60 oC and 30 ml of ethanol was added and stirred at for 4 hrs. The product was stripped for 2 hrs at 80 oC using a short path distillation head, nitrogen sparge and an overhead stirrer at 50 torr. The resulting clear light brown fluid exhibited a viscosity of 107.1 cP at 25 oC

### Synthetic Example 10: Preparation of D50

TMS protected butenoic acid (BATMS) (36.04 g) was added in a 500 mL 4-neck flask equipped with a N2 blanket, condenser and a thermocouple. A hydride functional silicone with the structure H(CH3)2SiO[Si(CH3)2)O]50Si(CH3)2H (214.06 g) was added slowly using an addition funnel. The temperature controller was set to 85 oC. The reaction was catalyzed with Karstedt's catalyst (10 ppm of Pt). After 1.5 hr the reaction was completed as indicated using a caustic fermentation tube. The reaction then cooled to 60 oC and 20 ml of ethanol was added and stirred at for 4 hrs. The product was stripped for 2 hrs at 80 oC using a short path distillation head, nitrogen sparge and an overhead stirrer at 50 torr. The resulting clear light brown fluid exhibited a viscosity of 177 cP at 25 oC

### Synthetic Example 11: Preparation of D100

TMS protected butenoic acid (BATMS) (12.62 g) was added in a 500 mL 4-neck flask equipped with a N2 blanket, condenser and a thermocouple. A hydride functional silicone with the structure H(CH3)2SiO[Si(CH3)2)O]100Si(CH3)2H (186.81 g) was added slowly using an addition funnel. The temperature controller was set to 85 oC. The reaction was catalyzed with Karstedt's catalyst (10 ppm of Pt). After 18 hrs the reaction was completed as indicated using a caustic fermentation tube. The reaction then cooled to 60 oC and 30 ml of ethanol was added and stirred at for 4 hrs. The product was stripped for 2 hrs at 80 oC using a short path distillation head, nitrogen sparge and an overhead stirrer at 50 torr. The resulting product was a clear colorless fluid.

### Synthetic Example 12: Preparation of 578-34 MD73D^{∗}27M

TMS protected butenoic acid (BATMS) (42.6 g) was added in a 250 mL 4-neck flask equipped with a N2 blanket, condenser and a thermocouple. A hydride functional silicone with the structure (CH3)3SiO[Si(CH3)2)O]73[Si(CH3)(H)O]27Si(CH3)3 (57.8 g) was added slowly using an addition funnel. The temperature controller was set to 85 oC. The reaction was catalyzed with Karstedt's catalyst (10 ppm of Pt). After 6 hrs the reaction was recatalyzed with another 10 ppm Pt and stirred for 24hrs. The reaction was completed as indicated using a caustic fermentation tube. The reaction then cooled to 60 oC and 100 g of ethanol was added and stirred at for 4 hrs. The product was stripped for 4 hrs at 130 oC using a short path distillation head, nitrogen sparge and an overhead stirrer at 50 torr. Diatomaceous earth (1.7 g) was added and the solution was filtered through a 5 µM filter. The resulting material was a clear colorless fluid.

### Carboxylic Acid Functional Silicone (CAFS) Microemulsion

**TABLE 1**

| | Chemical Name | % |
|---|---|---|
| A | Synthetic Example 1 (a) | 25 |
| | Dipropylene Glycol (surfactant b) | 5 |
| | Trideceth-6 (surfactant b) | 18.8 |
| B | Sodium Chloride | 1 |
| | Water (c) | 40.3 |
| C | Sodium Hydroxide | 0.2 |
| | Water (c) | q.s. to 100 |

1. Part A was mixed by mixing Synthetic Example 1 (a) and the two (b) surfactants together and heated to 50 °C, the mixing was continued for 15 minutes at 300 rpm using an overhead stirrer.
2. Part B: A solution of sodium chloride in water was mixed, and, added in four parts with mixing to part A until completely incorporated.
3. Part C: A solution of sodium hydroxide in water was mixed and then added to the A+B mixture. The mixing speed was reduced after 15 minutes and the emulsion was allowed to cool to room temperature. The pH was checked and adjusted by citric acid to 6.5-7.

### Negative Control Emulsion

**TABLE 2**

| | Chemical Name | % |
|---|---|---|
| A | Dipropylene Glycol (surfactant b) | 5 |
| | Trideceth-6 (surfactant b) | 18.8 |
| B | Sodium Chloride | 1 |
| | Water (c) | 40.3 |
| C | Sodium Hydroxide | 0.2 |
| | Water (c) | q.s. to 100 |

1. For part A. The two surfactants (b) were combined together and heated to 50 °C, and mixed for an additional 15 minutes at 300 rpm using an overhead stirrer.
2. For part B. A solution of sodium chloride in water was mixed, and add in four parts with mixing to part A until completely incorporated.
3. For part C. A solution of sodium hydroxide in water was combined with the A+B mixture. The mixing speed was reduced after 15 minutes and the emulsion was allowed to cool to room temperature. The pH was checked and adjusted by citric acid to 6.5-7.

### HAIR APPLICATION

### Application Example 1: Leave on Hair Conditioner for Virgin Latin Curly Hair Leave on Conditioner Formulation 1 (F1)

**TABLE 3**

| Component | % |
|---|---|
| CAFS Microemulsion | 1.2 |
| Fragrance 6113578 from Bell Flavors & Fragrances Inc | 0.1 |
| Water | q.s. to 100 |

A 1.2% CAFS microemulsion and 0.1% fragrance were mixed together for 5 minutes with a magnetic stirrer and then diluted with water to reach 100%.

### Comparative Formulation 1 (C1) - Negative Control Formulation

**TABLE 4**

| Component | % |
|---|---|
| Negative Control Emulsion | 1.2 |
| Fragrance 6113578 from Bell Flavors & Fragrances Inc | 0.1 |
| Water | q.s. to 100 |

1.2% negative control emulsion and 0.1% fragrance were mixed together for 5 minutes with a magnetic stirrer and then diluted with water to reach 100%.

Leave-on Treatment: 2 separate samples of, virgin latin hair, were used, one was dipped in leave on conditioner formulation F1 for one minute and the other was dipped in the C1 formulations for 1 minute. The extra from each was squeezed out and the hair was blow dried at low heat for 10-15 minutes until fully dry. The evaluation was done after staying in room condition open air overnight, but within 24 hours.

Evaluation: A panel study was conducted using 8 trained panelists . Each panelist were asked to smell coffee prior to and between smelling tresses to clear their nose. Each panelist smelled the tresses and ranked tresses in order of strength of fragrance smell.

Results: 8 out of 8 panelists picked the carboxylic acid functional silicone containing formulation F1 treated hair and none of the panelists picked the negative control C1 treated hair.

### Application Example 2: Stability of Emulsified Fragrance and Fragrance Retention Effect of Leave on Conditioner for Virgin Latin Curly Hair

### Leave on Conditioner Formulation 1 (F1)

**TABLE 5**

| Component | % |
|---|---|
| CAFS Microemulsion | 1.2 |
| Fragrance 6113578 from Bell Flavors & Fragrances Inc | 0.1 |
| Water | q.s. to 100 |

1.2% CAFS microemulsion and 0.1% fragrance were mixed together for 5 minutes with a magnetic stirrer and then diluted with water to reach 100%.

### Comparative Formulation 2 (C2) - SME253 containing formulation

**TABLE 6**

| Component | % |
|---|---|
| SME 253 (20% silicone active)^{∗} | 1.5 |
| Negative Control Emulsion | 1.2 |
| Fragrance 6113578 from Bell Flavors & Fragrances Inc | 0.1 |
| Water | q.s. to 100 |

| | |
|---|---|
| ^{∗}SME 253 is an amine functional silicone microemulsion containing 20% silicone solids with an average particle size of less than 20 nanometers which contains amododimethicone, C11-15 Pareth-7, Laureth-9, glycerin and trideceth-12 and is available from Momentive. | |

### Comparative Formulation 3 (C3) - Silsoft Silk containing formulation

**TABLE 7**

| Component | % |
|---|---|
| Silsoft Silk (36% silicone active)^{∗} | 0.83 |
| Negative Control Emulsion | 1.2 |
| Fragrance 6113578 from Bell Flavors & Fragrances Inc | 0.1 |
| Water | q.s. to 100 |

| | |
|---|---|
| ^{∗}Silsoft Silk amino silicone quat is a non-yellowing, 36% active microemulsion of a patented quaternized silicone terpolymer (silicone quat) containing silicone quaternium-18, trideceth-6, deceth-7, cocamidopropyl betaine and dipropylene glycol and is available from Momentive. | |

1.5% SME253 or 0.83% Silsoft Silk, 1.2% negative control emulsion and 0.1% fragrance were mixed together for 5 minutes with magnetic stirrer and then diluted with water to reach 100%.

### Stability of Emulsified Fragrance

The carboxylic acid functional silicone formulation F1 was clear and stable. The SME253 and Silsoft Silk formulations C2 and C3 were white and not stable after several days. If the carboxylic acid functional silicone surfactant package negative control emulsion was not included in the SME253 and Silsoft Silk formulation, the fragrance was not able to be emulsified.

### Fragrance Retention Effect

Leave-on Treatment: Three separate samples of Virgin Latin hair, were used. One was dipped in the leave on conditioner formulation F1 for one minute, another was dipped in the leave on conditioner formulation C2 for one minute and the final samples was dipped in leave on conditioner formulation C3 for 1 minute. The extra from each sample was squeezed out and the hair samples were blow dried at low heat for 10-15 minutes until fully dry. The hair samples were then put in a room conditioner in open air. The evaluation was done 1-3 hours after drying and overnight within 24 hours.

Evaluation: A panel study was conducted using 6 trained panelists. Each panelist were asked to smell coffee prior to and between smelling tresses to clear their nose. Each panelist smelled the tresses and ranked tresses in order of strength of fragrance smell.

Results: 6 of 6 panelists picked the carboxylic acid functional silicone containing conditioner F1 over the SME253 conditioner C2 and the Silsoft Silk conditioner C3.

### Application Example 3: Fragrance Retention Effect for another Fragrance in Leave on Conditioner for Virgin Latin Curly Hair

### Leave on Conditioner Formulation 1 (F2)

**TABLE 8**

| Component | % |
|---|---|
| CAFS Microemulsion | 1.2 |
| Fragrance Floral Fruity R13-5175 from Robertet, Inc. | 0.1 |
| Water | q.s. to 100 |

A 1.2% CAFS microemulsion and 0.1% fragrance were mixed together for 5 minutes with a magnetic stirrer and then diluted with water to reach 100%.

### Comparative Formulation 1 (C4) - Element 14 PDMS 100 containing formulation

**TABLE 9**

| Component | % |
|---|---|
| Element 14 PDMS 100^{∗} | 0.3 |
| Negative Control Emulsion | 1.2 |
| Fragrance Floral Fruity R13-5175 from Robertet, Inc. | 0.1 |
| Water | q.s. to 100 |

| | |
|---|---|
| **[0096]** ^{∗}Element 14 PDMS 100 is dimethicone with kinematic viscosity of 100 cst; SME 253 is amodimethicone microemulsion with 20% silicone active and Silsoft Silk is amino silicone quat microemulsion with 36% silicone active, each of which are available from Momentive. | |

### Comparative Formulation 2 (C5) - SME253 containing formulation

**TABLE 10**

| Component | % |
|---|---|
| SME 253 (20% silicone active)^{∗} | 1.5 |
| Negative Control Emulsion | 1.2 |
| Fragrance Floral Fruity R13-5175 from Robertet, Inc. | 0.1 |
| Water | q.s. to 100 |

### Comparative Formulation 3 (C6) - Silsoft Silk containing formulation

**TABLE 11**

| Component | % |
|---|---|
| Silsoft Silk (36% silicone active)^{∗} | 0.83 |
| Negative Control Emulsion | 1.2 |
| Fragrance Floral Fruity R13-5175 from Robertet, Inc. | 0.1 |
| Water | q.s. to 100 |

A 0.3% Element 14 PDMS 100 or 1.5% SME253 or 0.83% Silsoft Silk, 1.2% negative control emulsion and 0.1% fragrance were mixed together for 5 minutes with magnetic stirrer and then diluted with water to reach 100%.

Leave-on Treatment: 3 samples of Virgin Latin hair were used. Each sample was dipped in only one of the leave on conditioner formulations F2, C4, C5 or C6 for 1 minute. The extra from each sample was squeezed out and the hair was blow dried at low heat for 10-15 minutes until fully dry. The evaluation was done after the samples stay in room condition open air 1-2 hours and overnight but within 24 hours.

Evaluation: A panel study was conducted using 6 trained panelists . Each panelist were asked to smell coffee prior to and between smelling tresses to clear their nose. Each panelist smelled the tresses and ranked tresses in order of strength of fragrance smell.

Results: 6 out of 6 panelists picked the carboxylic acid functional silicone containing formulation F2 treated hair over the Element 14 PDMS 100, SME253 and Silsoft Silk containing comparative formulation C4, C5 and C6 1-2 hours after drying and overnight but within 24 hours after drying.

### Application Example 4: Hair Serum for Peroxide Damaged Latin Curly Hair Hair Serum Formulation 1- Methocell Thickener Fragrance 6113578 from Bell

### Hair Serum Formulation 1 (F3)

**TABLE 12**

| Component | % |
|---|---|
| CAFS Microemulsion | 4 |
| Fragrance 6113578 from Bell Flavors & Fragrances Inc | 0.25 |
| Methocell E4M Premium AMC from the Dow Chemical Company | 0.5 |
| Water | q.s. to 100 |

A 4% CAFS microemulsion and 0.25% fragrance were mixed together for 5 minutes with a magnetic stirrer and then a 3% Methocell thickened water and additional water were added to reach 100% with mixing. The result was a clear formulation.

### Comparative Hair Serum Formulation 1 (C7) - Negative Control Serum Formulation

**TABLE 13**

| Component | % |
|---|---|
| Fragrance 6113578 from Bell Flavors & Fragrances Inc | 0.25 |
| Methocell E4M Premuium AMC from the Dow Chemical Company | 0.5 |
| Water | q.s. to 100 |

0.25% fragrance was mixed with a 3% Methocell thickened water and additional water were to reach 100%. This is a white formulation.

### Comparative Hair Serum Formulation 2 (C8): Fructis Sleek & Shine Anti-Frizz Serum by Garnier Fructis (decamethylcyclopentasiloxane based formulation).

Hair peroxide damage protocol: Hair was assaulted twice with a 50 ml of a solution containing 6% H₂O₂ and 0.05% NaOH in water for 25 minutes each. The peroxide solutions were changed in between. 10% sodium laureth sulfate (SLES) was used to wash away the peroxide.

Serum Treatment: 3 separate samples of 4 g of peroxide damaged Latin curly hair were used. The hair serums F3, C7 and C8 were each separately rubbed in the amount 0.5g into only one of the 3 tresses, and allowed to air dry. Then the hair was put in a room conditioner in open air overnight within 24 hours.

Evaluation: A panel study was conducted using 4 trained panelists. Each panelist were asked to smell coffee prior to and between smelling tresses to clear their nose. Each panelist smelled the tresses and ranked tresses in order of strength of fragrance smell.

Results: 4 of 4 panelists picked the carboxylic acid functional silicone containing serum F3 over the negative control serum C7 and the commercial benchmark serum C8.

### Hair Serum Formulation 2 Comparative Tests - Xanthan Gum thickener Fragrance 90-2653-41 from Lebermuth company. Inc.

### Hair Serum Formulation 2 (F4)

**TABLE 14**

| Component | % |
|---|---|
| CAFS Microemulsion | 12 |
| Fragrance 90-2653-41 from the Lebermuth Company, Inc. | 0.25 |
| Xanthan Gum Keltrol CG-SFT from CP Kelco US, Inc. | 1 |
| Glydant Plus (DMDM Hydantoin, Iodopropynyl Butylcarbamate) from Lonza Group Ltd. | 0.25 |
| Water | q.s. to 100 |

12% CAFS microemulsion and 0.25% fragrance was mixed together for 5 minutes with magnetic stirrer and then add 2% Xanthan gum thickened water, additional water and Glydant Plus to reach 100% with mixing. This was a clear formulation.

### Comparative Hair Serum Formulation 2 (C8): Fructis Sleek & Shine Anti-Frizz Serum by Garnier Fructis (D5 based formulation).

### Comparative Hair Serum Formulation 3 (C9) - Negative Control Serum Formulation

**TABLE 15**

| Component | % |
|---|---|
| Fragrance 90-2653-41 from the Lebermuth Company, Inc. | 0.25 |
| Xanthan Gum Keltrol CG-SFT from CP Kelco US, Inc. | 1 |
| Glydant Plus (DMDM Hydantoin, Iodopropynyl Butylcarbamate) from Lonza Group Ltd. | 0.25 |
| Water | q.s. to 100 |

0.25% fragrance was mixed with 2% Xanthan gum thickened water, additional water and Glydant plus to reach 100%. The formulation was white in appearance.

Serum Treatment: The hair serum F4, C8 and C9 were each independently rubbed into separate peroxide damaged Latin curly hair samples in the amount 0.5g per 4g tress, and allowed to air dry. Evaluation was done after putting hair in a room conditioner at open air overnight but within 24 hours.

Evaluation: A panel study was conducted using 4 trained panelists. Each panelist were asked to smell coffee prior to and between smelling tresses to clear their nose. Each panelist smelled the tresses and ranked tresses in order of strength of fragrance smell.

Results: 4 of 4 panelists picked the carboxylic acid functional silicone containing serum F4 over negative control serum C8 and commercial benchmark serum C9. And F4 had better anti-frizz effect than the commercial benchmark anti-frizz formulation C9.

### Application Example 5: Rinse-off Conditioner for Virgin Latin Curly Hair Rinse-off Conditioner Base

**TABLE 16**

| | Chemical Name | % |
|---|---|---|
| A | Water | Up to 90 |
| | Lactic Acid | 0.6 |
| B | Amidet APA-22 (Behenamidopropyl Dimethylamine) from Kao Corporation | 2.2 |
| C | Kalcol 6850 (Cetostearyl alcohol) from Kao Corporation | 4.4 |

1. For part A. Lactic acid and water were mixed and heated to 80 °C.
2. Part B was added to part A all at once, and mixed with mechanical stirring for 1-3 hours at 80 °C to provide a homogeneous mixture.
3. Part C was added to the mixture of A and B all at once and stirred at 80 °C for 30 minutes to 1 hour till completely melted a homogeneous mixture was obtained.
4. The mixture was remove from heating and stirred continuously till reaching room temperature.

### Rinse-off Conditioner Formulation F5

**TABLE 17**

| Component | % |
|---|---|
| CAFS Microemulsion | 4 |
| Fragrance 6113578 from Bell Flavors & Fragrances Inc | 0.1 |
| Rinse-off Conditioner Base | 90 |
| Water | q.s. to 100 |

### Comparative Rinse-off Conditioner Formulation 1 (C10) - Contains SME253

**TABLE 18**

| Component | % |
|---|---|
| SME253 | 5 |
| Fragrance 6113578 from Bell Flavors & Fragrances Inc | 0.1 |
| Rinse-off Conditioner Base | 90 |
| Water | q.s. to 100 |

### Comparative Rinse-off Conditioner Formulation 2 (C11) - Contains Silsoft Silk

**TABLE 19**

| Component | % |
|---|---|
| Silsoft Silk | 2.78 |
| Fragrance 6113578 from Bell Flavors & Fragrances Inc | 0.1 |
| Rinse-off Conditioner Base | 90 |
| Water | q.s. to 100 |

### Comparative Rinse-off Conditioner Formulation 3 (C12) - Negative Control

**TABLE 20**

| Component | % |
|---|---|
| Fragrance 6113578 from Bell Flavors & Fragrances Inc | 0.1 |
| Rinse-off Conditioner Base | 90 |
| Water | q.s. to 100 |

For each formulation, the components were added together, and mixed with a centrifugal homogenizer at 3000 rpm for 2 minutes.

Rinse-off Treatment: 1.2g of rinse off conditioner F5, C10, C11 and C12 were each separately applied to only one of 4 separate samples of 4g virgin Latin curly hair. The specific formulations were rubbed in evenly and washed with warm water. The hair was then blow dried at low heat for 30 minutes to fully dry. Then the hair was put in a room conditioner at open air. Evaluation was done 1-3 hours after drying and 24 hours after drying.

Evaluation: A panel study was conducted using 5 trained panelists. Each panelist were asked to smell coffee prior to and between smelling tresses to clear their nose. Each panelist smelled the tresses and ranked tresses in order of strength of fragrance smell.

Results: 5 of 5 panelists picked the carboxylic acid functional silicone containing rinse-off conditioner F5 over negative control rinse-off conditioner C12 and SME253 and Silsoft Silk containing rinse-off conditioner C10 and C11 for both 1-3 hours and 24 hours after drying. After 3 days, only F5 had a fragrance scent.

### Application Example 6: Other Hair Care Applications

### Clear Shampoo Formulation

**TABLE 21A**

| | Component | % |
|---|---|---|
| A | Sodium Laureth Sulfate | 9 |
| | Cocamidopropyl Betaine | 3 |
| B | Lauryl glycoside | 1.85 |
| C | Carbopol Aqua SF-1 from Lubrizol, Inc | 7.31 |
| D | CAFS Microemulsion | 4 |
| E | Triethanolamine | 1.1 |
| | Water | q.s. to 100 |

| | | |
|---|---|---|
| Part A: Components of part A were mixed together with overhead mechanical stirrer at 600 rpm for 10 minutes. Part B was added to part A, and stirred with the mechanical stirrer at 600 rpm for 10 minutes. Part A+B was obtained. Part C was added to part A+B and was stirred with the mechanical stirrer at 600 rpm for 30 minutes. Part A+B+C was obtained. Part D was then added to part A+B+C and then stirred for 30 minutes with the mechanical stirrer at 600 rpm for 30 minutes. Part A+B+C+D was obtained. Part E was then added to part A+B+C+D and was stirred with the mechanical stirrer for 30 minutes at 600 rpm to homogeneous. pH was adjusted to 7 with NaOH. | | |

### Carboxylic Acid Functional Silicone (CAFS) Emulsion I

**TABLE 21B**

| Chemical Name | % |
|---|---|
| Synthetic Example 11 | 20 |
| Trideceth-8 | 12 |
| Water | q.s. to 100 |

Synthetic Example 11 and surfactant Trideceth-8 was mixed for 15 minutes at 300 rpm using an overhead stirrer. Water was added to the mixture and the mixing continued for another 15 minutes. Sodium hydroxide was then used to adjust the pH to 6.5-7.

### Pearl Shampoo with CAFS Emulsion I

**TABLE 22**

| | Component | % |
|---|---|---|
| A | Sodium Laureth Sulfate | 12 |
| | Cocamidopropyl Betaine | 3 |
| B | Ethylene Glycol Distearate | 1 |
| | Water | 10 |
| C | Cocamide Monoethanolamide | 1 |
| | Water | 10 |
| D | Polyquaternium-6 | 0.06 |
| | ACULYN^{™} 38 from the Dow Chemical Company (10% active) | 3 |
| E | CAFS Emulsion I | 2 |
| F | Water | q.s. to 100 |

| | | |
|---|---|---|
| Part A: Components of part A were mixed together with overhead mechanical stirrer at 600 rpm for 10 minutes. Part B: 1 g ethylene glycol distearate and 10 g water was mixed together with magnetic stirrer at 200 rpm for 15 minutes. Part C: 1 g cocamide monoethanolamide and 10 g water was mixed together with magnetic stirrer at 200 rpm for 15 minutes. Components of part D were added to part A and stirred with overhead mechanical stirrer at 600 rpm for 10 minutes. Part A+D was obtained. Part B was added to part A+D, and stirred for 10 minutes at 600 rpm with mechanical stirrer. Part A+D+B was obtained. Part C was added to part A+D+B, and was stirred for 10 minutes at 600 rpm with mechanical stirrer. Part A+D+B+C was obtained. Part E was then added to part A+D+B+C, and was stirred for 15 minutes at 600 rpm with mechanical stirrer. Part A+D+B+C+E was obtained. Part F was added last to above and stirred for 15 minutes at 600 rpm with mechanical stirrer. | | |

### Pearl Shampoo with CAFS Emulsion I Non-Sulfate Version

**TABLE 23**

| | Component | % |
|---|---|---|
| A | Sodium Lauryl Sulfoacetate + Disodium Laureth Sulfosuccinate | 10.6 |
| | Cetyl Betaine | 3.3 |
| B | Cocamide Monoethanolamide | 1.5 |
| | Water | 10 |
| C | Hydroxypropyl Methylcellulose | 1.5 |
| | Water | 10 |
| D | Ethylene Glycol Distearate | 1.5 |
| | Water | 10 |
| E | Polyquaternium-10 | 0.15 |
| F | CAFS Emulsion I | 2 |
| G | Water | q.s. to 100 |

| | | |
|---|---|---|
| Part A: Components of part A were mixed together with overhead mechanical stirrer at 600 rpm for 10 minutes. Part B: 1.5 g cocamide monoethanolamide was mixed with 10 g 45 °C water by magnetic stirring at 200 rpm for 30 minutes. Part C: 1.5 g hydroxypropyl methylcellulose powder was slowly added to 10 g 45 °C water with magnetic stirring at 200 rpm. The total stirring time was 30 minutes. Part D: 1.5 g Ethylene glycol distearate powder was slowly added to 10 g 45 °C water with magnetic stirring at 200 rpm. The total stirring time was 30 minutes. Part B was slowly added to part A with mechanical stirring at 600 rpm for 5 minutes. Part A+B was obtained. Part C was slowly added to part A+B with mechanical stirring at 600 rpm for 5 minutes. Part A+B+C was obtained. Part D was slowly added to part A+B+C with mechanical stirring at 600 rpm for 5 minutes. Part A+B+C+D was obtained. Part E was added to part A+B+C+D with mechanical stirring at 600 rpm for 10 minutes. Part A+B+C+D+E was obtained. Part F was then added to above mixture, mechanically stirred for 15 minutes at 600 rpm. Part G was then added after part F and stirred at 600 rpm for 30 minutes. | | |

### Anti-Frizz Shampoo

**TABLE 24**

| | Component | % |
|---|---|---|
| A | Water | 35 |
| | PEG-120 Methyl Glucose Dioleate | 2 |
| B | Water | 15.25 |
| | Sodium Laureth Sulfate | 9 |
| C | Dissodium EDTA | 0.1 |
| | Cocamidopropyl Betaine | 10 |
| | Polyquaternium-7 | 0.5 |
| | Decyl Glucoside | 1 |
| | Dexpanthenol | 1 |
| | Phenoxy ethanol | 0.5 |
| D | CAFS Microemulsion | 4 |
| | Tropicalism 18 from Givaudan S.A. | 0.65 |

| | | |
|---|---|---|
| Part A: Components of part A was mixed together with magnetic stirrer at 200 rpm for 15 minutes. Part B: Components of part B was mixed together with magnetic stirrer at 200 rpm for 15 minutes. Part B was added to part A and mixed for 15 minutes at 500 rpm with overhead mechanical stirrer. Components of part C were added to part A+B with mechanical stirring at 500 rpm for 1 hour. Components of part D were mixed together with magnetic stirrer at 200 rpm for 15 minutes and then added to part A+B+C. | | |

The whole mixture was then mixed with mechanical stirrer at 500 rpm for 30 minutes.

### Anti-Frizz Conditioner

**TABLE 25**

| | Component | % |
|---|---|---|
| A | Water | q.s. to 100 |
| | Cetrimonium Chloride | 2 |
| B | Disodium EDTA | 0.05 |
| | Cetearyl Alcohol | 4.5 |
| | Glyceryl Stearate | 1 |
| | Behenamidopropyl Dimethylamine | 0.5 |
| | Tocopheryl Acetate | 0.5 |
| C | Prodew 500 from Ajinomoto Co., Inc. | 0.5 |
| D | DMDM Hydantoin | 0.5 |
| E | CAFS Microemulsion | 4 |
| | Tropicalism 18 from Givaudan S.A. | 0.65 |

| | | |
|---|---|---|
| Part A: Components of part A was heated to 85 °C and mixed together with magnetic stirrer at 200 rpm for 15 minutes. Part B: Components of part B was heated to 85 °C mixed together with magnetic stirrer at 200 rpm for 15 minutes. Part B was added to part A and mixed for 15 minutes at 500 rpm with overhead mechanical stirrer at 85 °C. Part C and D were added to part A+B and mixed with mechanical stirrer at 500 rpm for 30 minutes. Components of part E was mixed together with magnetic stirrer at 200 rpm for 15 minutes and then added to part A+B+C+D. The mixture was then stirrer with mechanical stirrer at 500 rpm for 30 minutes. | | |

### SKIN APPLICATION

### Application Example 7: Moisturizing Creme Body Wash with Natural Oil

### Moisturizing Creme Body Wash with Natural Oil Formulation, CAFS Microemulsion containing Formulation (F6)

**TABLE 26**

| | Component | % |
|---|---|---|
| A | Sodium Laureth Sulfate | 11 |
| | Cocamidopropyl Betaine | 1.8 |
| B | Sunflower Oil | 18 |
| | Glycerin | 5 |
| C | Fragrance R15-2331 from Robertet, Inc. | 1.5 |
| | CAFS Microemulsion | 12 |
| D | Jaguar Excel from Solvay Novecare | 0.3 |
| E | Xanthan Gum Keltrol CG-SFT from CP Kelco US, Inc. | 1.5 |
| | Water | q.s. to 100 |

### Moisturizing Creme Body Wash with Natural Oil Formulation, CAFS containing Formulation (F7)

**TABLE 27**

| | Component | % |
|---|---|---|
| A | Sodium Laureth Sulfate | 11 |
| | Cocamidopropyl Betaine | 1.8 |
| B | Sunflower Oil | 18 |
| | Glycerin | 5 |
| C | Fragrance R15-2331 from Robertet, Inc. | 1.5 |
| | Synthetic Example 1 | 3 |
| D | Jaguar Excel from Solvay Novecare | 0.3 |
| E | Xanthan Gum Keltrol CG-SFT from CP Kelco US, Inc. | 1.5 |
| | Water | q.s. to 100 |

### Moisturizing Creme Body Wash with Natural Oil Formulation, pH4 CAFS containing Formulation (F8)

**TABLE 28**

| | Component | % |
|---|---|---|
| A | Sodium Laureth Sulfate | 11 |
| | Cocamidopropyl Betaine | 1.8 |
| B | Sunflower Oil | 18 |
| | Glycerin | 5 |
| C | Fragrance R15-2331 from Robertet, Inc. | 1.5 |
| | Synthetic Example 1 | 3 |
| D | Jaguar Excel from Solvay Novecare | 0.3 |
| E | Xanthan Gum Keltrol CG-SFT from CP Kelco US, Inc. | 1.5 |
| | Water | q.s. to 100 |

### Comparative Creme Body Wash with Natural Oil Formulation 1 (C13) - Element 14 PDMS 100 containing Formulation

**TABLE 29**

| | Component | % |
|---|---|---|
| A | Sodium Laureth Sulfate | 11 |
| | Cocamidopropyl Betaine | 1.8 |
| B | Sunflower Oil | 18 |
| | Glycerin | 5 |
| C | Fragrance R15-2331 from Robertet, Inc. | 1.5 |
| | Element 14 PDMS 100 | 3 |
| D | Jaguar Excel from Solvay Novecare | 0.3 |
| E | Xanthan Gum Keltrol CG-SFT from CP Kelco US, Inc. | 1.5 |
| | Water | q.s. to 100 |

For each formulation, Part A was mixed with mechanical stirrer for 5 minutes. Then part B was added to part A and stirred for another 5 minutes to get part A+B. For formulation F6, Part C was mixed separately with magnetic stirrer for 5 minutes. Part C was then added to part A+B and mechanically stirred for 5 minutes. For formulation F7 and C13, part C was directly added to part A+B and mechanically stirred for 5 minutes. Part D was slowly added into room temperature water with magnetic stirring in 1 hour to get fully dispersed. Part E was slowly added into room temperature water with mechanical stirring in 30 minutes to get fully dispersed. Fully dispersed part D and part E was added into part A+B+C with compensate water to 100%, and stirred by mechanical stirrer for 30 minutes to get the final formulation. For pH4 CAFS containing formulation F8, the final formulation was adjusted to pH4 with citric acid. Other formulations were adjusted to pH7 with sodium hydroxide.

Body wash Treatment: Left and right forearms were wet under 42 °C water faucet. Then 1 mL of the body wash F6 and C13 was applied to left and right forearm. After that, both forearms were lathered for 30 seconds from wrist to elbow. Both forearms were then rinsed by 42 °C water under a running faucet without finger touch.

Evaluation: A panel study was conducted using trained panelists at 0 hour and 4 hours after washing.

7 panelists were used to compare Synthetic Example 1 CAFS containing formulation F7 and PDMS 100 containing formulation C13; 5 panelists were used to compare CAFS microemulsion containing formulation F6 and PDMS 100 containing formulation C13; For these two sets of comparison, panelists were asked to smell the forearms and give a score of 0 to 5, 0 as no smell and 5 as very strong smell.

7 panelists were used to compare CAFS microemulsion containing formulation F6 and CAFS containing formulation F7; 7 panelists were used to compare Synthetic Example 1 CAFS containing formulation F7 and pH4 CAFS containing formulation F8. For these two sets of comparison, panelists were asked to smell the forearms and tell which one has stronger smell.

In addition, the CAFS microemulsion containing formulation F6, Synthetic Example 1 CAFS containing formulation F7 and PDMS 100 containing formulation C13 were applied as rinse off conditioner on virgin Latin curly hair and evaluated by 6 panelists at 0 hour and 4 hours after washing. Panelists were asked to rank the hair tresses by strength of smell.

### Results:

### 7 Panelists Comparison of Synthetic Example 1 CAFS containing Formulation F7 and PDMS 100 containing Formulation C13

**TABLE 30**

| Time after washing | Synthetic Example 1 CAFS containing F7 Score | F7 Standard Deviation | PDMS 100 containing C13 Score | C13 Standard Deviation | p value |
|---|---|---|---|---|---|
| 0 Hour | 4.2 | 0.84 | 1.8 | 0.84 | 0.00018 |
| 4 Hours | 3.14 | 0.69 | 1 | 0.58 | 0.00006 |

### 5 Panelists Comparison of CAFS Micromulsion containing Formulation F6 and PDMS 100 containing Formulation C13

**TABLE 31**

| Time after washing | CAFS micro emulsion containing F6 Score | F6 Standard Deviation | PDMS 100 containing C13 Score | C13 Standard Deviation | p value |
|---|---|---|---|---|---|
| 0 Hour | 4 | 0.71 | 2.8 | 0.84 | 0.045 |
| 4 Hours | 2.75 | 0.5 | 1.5 | 0.58 | 0.008 |

### 7 Panelists Comparison of CAFS microemulsion containing Formulation F6 and Synthetic Example 1 CAFS containing Formulation F7

**TABLE 32**

| Time after washing | CAFS microemulsion containing F6 stronger | Synthetic Example 1 CAFS containing F7 stronger | The same |
|---|---|---|---|
| 0 Hour | 4 | 3 | 0 |
| 4 Hours | 1 | 4 | 2 |

| | | | |
|---|---|---|---|
| Note: The numbers in the table are number of panelists made the choice | | | |

### 7 Panelists Comparison of Synthetic Example 1 CAFS containing Formulation F7 at pH7 and pH4 Synthetic Example 1 CAFS containing Formulation F8

**TABLE 33**

| Time after washing | pH7 Synthetic Example 1 CAFS containing F7 stronger | pH4 Synthetic Example 1 CAFS containing F8 stronger | The same |
|---|---|---|---|
| 0 Hour | 5 | 1 | 2 |
| 4 Hours | 2 | 2 | 4 |

| | | | |
|---|---|---|---|
| Note: The numbers in the table are the number of panelists making the choice. | | | |

From table 23 (comparison of Synthetic Example 1 CAFS containing formulation F7 and PDMS 100 containing formulation C13), table 24 (comparison of CAFS microemulsion containing formulation F6 and PDMS 100 containing formulation C13) and table 25 (comparison of CAFS microemulsion containing formulation F6 and Synthetic Example 1 CAFS containing formulation F7), we can see that both Synthetic Example 1 CAFS and CAFS microemulsion have significant fragrance retention effect in the body wash formulation. In body wash, Synthetic Example 1 CAFS has slightly stronger fragrance retention effect than that of CAFS microemulsion. From table 26, we see that pH4 Synthetic Example 1 CAFS formulation has almost the same fragrance retention effect as that of the pH7 Synthetic Example 1 CAFS formulation. Since pH4 is mostly used for personal care formulations, working almost equivalently well at pH4 for fragrance retention is a strong plus for carboxylic silicone material in personal care fragrance retention applications.

In addition, we tried to compare the CAFS microemulsion containing formulation F6, Synthetic Example 1 CAFS containing formulation F7 and PDMS 100 containing formulation C13 as rinse off conditioner on virgin Latin curly hair and evaluated at 0 hour and 4 hours after washing. At 0 and 4 hours, all 6 panelists ranked the hair samples with strongest smell to weakest smell as CAFS microemulsion containing formulation F6 > Synthetic Example 1 CAFS containing formulation F7 > PDMS 100 containing formulation C13. So for hair applications, CAFS microemulsion is needed.

### Application Example 8: Simple Body Wash without Natural Oil or Glycerin Body Wash Formulation, CAFS Microemulsion containing Formulation (F9)

**TABLE 34**

| | Component | % |
|---|---|---|
| A | Sodium Laureth Sulfate | 11 |
| | Cocamidopropyl Betaine | 1.8 |
| B | Fragrance R15-2331 from Robertet, Inc. | 1.5 |
| | CAFS Microemulsion | 12 |
| C | Jaguar Excel from Solvay Novecare | 0.3 |
| D | Xanthan Gum Keltrol CG-SFT from CP Kelco US, Inc. | 1.5 |
| | Water | q.s. to 100 |

### Comparative Body Wash Formulation. Element 14 PDMS 100 emulsion containing Formulation (C14)

**TABLE 35**

| | Component | % |
|---|---|---|
| A | Sodium Laureth Sulfate | 11 |
| | Cocamidopropyl Betaine | 1.8 |
| B | Fragrance R15-2331 from Robertet, Inc. | 1.5 |
| | Element 14 PDMS 100 | 3 |
| | Negative Control Emulsion | 12 |
| C | Jaguar Excel from Solvay Novecare | 0.3 |
| D | Xanthan Gum Keltrol CG-SFT from CP Kelco US, Inc. | 1.5 |
| | Water | q.s. to 100 |

### Body Wash Formulation, pH4 CAFS Microemulsion containing Formulation (F10)

**TABLE 36**

| | Component | % |
|---|---|---|
| A | Sodium Laureth Sulfate | 11 |
| | Cocamidopropyl Betaine | 1.8 |
| B | Fragrance R15-2331 from Robertet, Inc. | 1.5 |
| | CAFS Microemulsion | 12 |
| C | Jaguar Excel from Solvay Novecare | 0.3 |
| D | Xanthan Gum Keltrol CG-SFT from CP Kelco US, Inc. | 1.5 |
| | Water | q.s. to 100 |

### Comparative Body Wash Formulation, pH4 Element 14 PDMS 100 emulsion containing Formulation (C15)

**TABLE 37**

| | Component | % |
|---|---|---|
| A | Sodium Laureth Sulfate | 11 |
| | Cocamidopropyl Betaine | 1.8 |
| B | Fragrance R15-2331 from Robertet, Inc. | 1.5 |
| | Element 14 PDMS 100 | 3 |
| | Negative Control Emulsion | 12 |
| C | Jaguar Excel from Solvay Novecare | 0.3 |
| D | Xanthan Gum Keltrol CG-SFT from CP Kelco US, Inc. | 1.5 |
| | Water | q.s. to 100 |

For each formulation, Part A was mixed with mechanical stirrer for 5 minutes. Part B was mixed separately with magnetic stirrer for 5 minutes. Part B was then added to part A and mechanically stirred for 5 minutes. Part C was slowly added into room temperature water with magnetic stirring in 1 hour to get fully dispersed. Part D was slowly added into room temperature water with mechanical stirring in 30 minutes to get fully dispersed. Fully dispersed part C and part D was added into part A+B with compensate water to 100%, and stirred by mechanical stirrer for 30 minutes to get the final formulation. For pH4 CAFS microemulsion containing formulation F10 and pH4 Element 14 PDMS 100 containing formulation C15, the final formulation was adjusted to pH4 with citric acid. Other formulations were adjusted to pH7 with sodium hydroxide.

Body wash Treatment: Left and right forearms were wet under 42 °C water faucet. Then 1 mL of the body wash F6 and C13 was applied to left and right forearm. After that, both forearms were lathered for 30 seconds from wrist to elbow. Both forearms were then rinsed by 42 °C water under a running faucet without finger touch.

Evaluation: A panel study was conducted using trained panelists at 0 hour and 4 hours after washing.

6 panelists were used to compare CAFS microemulsion containing formulation F9 and PDMS 100 emulsion containing formulation C14; 8 panelists were used to compare pH4 CAFS microemulsion containing formulation F10 and pH4 PDMS 100 emulsion containing formulation C15; Panelists were asked to smell the forearms and give a score of 0 to 5, 0 as no smell and 5 as very strong smell.

### Results:

### 6 Panelists Comparison of CAFS microemulsion containing Formulation F9 and PDMS 100 emulsion containing Formulation C14

**TABLE 38**

| Time after washing | CAFS microemulsion containing F9 Score | F9 Standard Deviation | PDMS 100 emulsion containing C14 Score | C14 Standard Deviation | p value |
|---|---|---|---|---|---|
| 0 Hour | 4.8 | 0.41 | 1.8 | 0.75 | 0.00006 |
| 4 Hours | 2.7 | 0.52 | 1 | 0 | 0.00049 |

### 8 Panelists Comparison of pH4 CAFS micromulsion containing Formulation F10 and PDMS 100 emulsion containing Formulation C15

**TABLE 39**

| Time after washing | pH4 CAFS microemulsion containing F10 Score | F10 Standard Deviation | pH4 PDMS 100 emulsion containing C15 Score | C15 Standard Deviation | p value |
|---|---|---|---|---|---|
| 0 Hour | 4.38 | 0.74 | 2.25 | 0.46 | 0.00003 |
| 4 Hours | 1.85 | 0.38 | 0.71 | 0.39 | 0.00005 |

From table 30 (comparison of CAFS microemulsion containing formulation F9 and PDMS 100 emulsion containing formulation C14) and table 31 (comparison of pH4 CAFS microemulsion containing formulation F10 and pH4 PDMS 100 emulsion containing formulation C15), we can see that CAFS microemulsion has significant fragrance retention effect at both pH4 and pH7. pH4 fragrance retention effect is slightly less than the pH7 fragrance retention effect.

### Application Example 9: Other Skin Care Formulations

### Body Mist

**TABLE 40**

| | Component | % |
|---|---|---|
| A | Glycerine | 5 |
| | Isododecane | 5 |
| | Fragrance Floral Fruity R13-5175 from Robertet, Inc. | 0.1 |
| | Synthetic Example 11 | 1 |
| | Vitamin E | 0.25 |
| B | Pemulen^{™} TR-1 Polymeric Emulsifier from Lubrizol Corporation | 0.25 |
| | Water | 25 |
| C | Trideceth-8 | 0.25 |
| | Water | q.s. to 100 |

| | | |
|---|---|---|
| Part A: components of part A were mixed together with an overhead mechanical stirrer at 500 rpm for 15 minutes. Part B: 0.25 g Pemulen TR-1 was mixed with 25 g 45 °C water for 30 minutes with magnetic stirrer at 200 rpm. Part B was then slowly added to part A with mechanical stirring at 500 rpm for 15 minutes. Get part A+B. Part C: 0.25 g Trideceth-8 was mixed with remaining water for 15 minutes with magnetic stirrer at 200 rpm. Part C was then added to part A+B and stirred with overhead mechanical stirrer for 15 minutes at 500 rpm. | | |

### Body Serum (water based)

**TABLE 41**

| | Component | % |
|---|---|---|
| A | Jojoba Oil | 3 |
| | Glycerine | 4 |
| | Isododecane | 2 |
| B | Fragrance Floral Fruity R13-5175 from Robertet, Inc. | 0.1 |
| | Synthetic Example 11 | 1 |
| | Vitamin E | 0.25 |
| C | Pemulen^{™} TR-1 Polymeric Emulsifier from Lubrizol Corporation | 0.25 |
| | Water | 25 |
| D | Water | q.s. to 100 |
| E | Sepigel from SEPPIC, Inc. | 0.25 |

| | | |
|---|---|---|
| Part A: components of part A were mixed together with an overhead mechanical stirrer at 500 rpm for 15 minutes. Components of part B was added to part A and stirred for 5 minutes at 500 rpm with overhead mechanical stirrer. Part C: 0.25 g Pemulen TR-1 was mixed with 25 g 45 °C water for 30 minutes with magnetic stirrer at 200 rpm. Part C was then added to part A+B, and stirred with mechanical stirrer for 15 minutes at 500 rpm. Part D the rest of water was added to A+B+C and stirred for 15 minutes at 500 rpm with overhead mechanical stirrer. Part E the Sepigel was added to A+B+C+D and stirred for 30 minutes at 500 rpm with overhead mechanical stirrer. | | |

### Body Lotion

**TABLE 42**

| | Component | % |
|---|---|---|
| A | Carbopol 1382 from Lubrizol, Inc dispersed in water at concentration of 0.5% | 20 |
| B | Steric Acid | 4 |
| | Cetyl Alcohol | 0.6 |
| | Water | 20 |
| C | Jojoba Oil | 3 |
| | Sunflower Oil | 3 |
| | Isopropyl Myristate | 1.5 |
| | Fragrance Floral Fruity R13-5175 from Robertet, Inc. | 0.1 |
| | Synthetic Example 11 | 1 |
| | Vitamin E | 0.25 |
| D | Glycerine | 3 |
| E | Water | q.s. to 100 |

| | | |
|---|---|---|
| Part A: Carbopol 1382 was slowly added into 45 °C with magnetic stirring at 200 rpm. It was totally stirred for 1 hour to get fully dispersed. Part B: All components of part B were mixed together with magnetic stirring for 30 minutes at 200 rpm. Part C: All components of part C were mixed together with an overhead mechanical stirrer at 50 rpm for 15 minutes. Part D was added to part C and stirred with mechanical stirrer at 500 rpm for 10 minutes. Part C+D was obtained. Part B was then slowly added to part C+D with mechanical stirrer at 500 rpm for 15 minutes. Part C+D+B was obtained. Part E the remaining water was added to part C+D+B and stirred 15 minutes with overhead mechanical stirrer for 15 minutes at 500 rpm. Part C+D+B+E was obtained. Part A was then added to part C+D+B+E and stirred for 30 minutes with mechanical stirrer at 500 rpm. | | |

### Body Serum, water/D5(Cyclopentasiloxane) based

**TABLE 43**

| | Component | % |
|---|---|---|
| A | D5 (Cyclopentasiloxane) | 13 |
| | Jojoba Oil | 3 |
| | Coconut Oil | 3 |
| | Isopropyl Myristate | 1.65 |
| | Silform 60A from Momentive Performance Materials, Inc | 2 |
| B | Xanthan Gum Keltrol CG-SFT from CP Kelco US, Inc. | 0.25 |
| | Water | 25 |
| C | Glycerine | 3 |
| | Fragrance Floral Fruity R13-5175 from Robertet, Inc. | 0.1 |
| | Synthetic Example 11 | 1 |
| | Vitamin E | 0.25 |
| D | Bentone Gel^{®} VS-5PC V from Elementis Specialties, Inc. | 2.5 |
| E | Water | q.s. to 100 |

| | | |
|---|---|---|
| Part A: Components of part A were mixed with overhead mechanical stirrer for 30 minutes at 500 rpm. Part B: Xanthan Gum powder was slowly added to 45 °C water with magnetic stirring at 200 rpm. The total stirring time was 1 hour. Components of part C were added to part A and stirred with mechanical stirrer for 15 minutes at 500 rpm. Part A+C was obtained. Part D was then added to part A+C, and stirred with mechanical stirrer at 500 rpm for 15 minutes. Part A+C+D was obtained. Part B was then mixed with part A+C+D with mechanical stirrer at 500 rpm for 15 minutes. Part A+C+D+B was obtained. Part E the remaining water was then added to part A+C+D+B and mixed with mechanical stirrer for 30 minutes at 500 rpm. | | |

### Non-Whitening Roll-on Antiperspirant

**TABLE 44**

| | Component | % |
|---|---|---|
| A | SF1550 from Momentive Performance Materials, Inc. | 2 |
| | Silsoft 034 from Momentive Performance Materials, Inc. | 1 |
| | Silsoft ETS from Momentive Performance Materials, Inc. | 3 |
| B | PPG-14 Buthyl Ether | 2 |
| | Sunflower Oil | 1 |
| | Steareth-21 | 3.78 |
| | Steareth-2 | 0.72 |
| C | Propylene Glycol | 8 |
| | Aluminum Chlorohydrate | 20 |
| | Glycerine | 4 |
| | Water | q.s. to 100 |
| D | Acnibio AP from Univar, Inc. | 0.5 |
| E | Caring 19 from Givaudan S.A. | 0.5 |
| | CAFS Microemulsion | 4 |

| | | |
|---|---|---|
| Part A: Components of part A were mixed with overhead mechanical stirrer for 30 minutes at 500 rpm. Part B: Components of part B were heated to 75 °C and mixed with magnetic stirrer at 200 rpm for 15 minutes. Part C: Components of part C were heated to 75 °C and mixed with magnetic stirrer at 200 rpm for 15 minutes. Part B was added to part C while hot with magnetic stirring at 200 rpm for 30 minutes. And then was cooled to 30 °C and added to part A. The mixture was mixed with mechanical stirrer for 30 minutes at 500 rpm. Part E: Components of part E were mixed with magnetic stirrer at 200 rpm for 15 minutes. Part D and part E were added to part A+B+C and mixed with mechanical stirrer at 500 rpm for 30 minutes. | | |

### Antiperspirant Aerosol

**TABLE 45**

| | Component | % |
|---|---|---|
| A | Silsoft DML from Momentive Performance Materials, Inc. | q.s. 100 |
| | Bentone Gel^{®} VS-5PC V from Elementis Specialties, Inc. | 9 |
| B | Aluminum Sesquichlorohydrate | 30 |
| C | Silshine 151 from Momentive Performance Materials, Inc. | 0.5 |
| | Silsoft Silicone Gel from Momentive Performance Materials, Inc. | 3 |
| | Isopropyl Palmitate | 10 |
| | Sensidin DO from Schulke & Mayr, Inc. | 0.5 |
| D | Men White 65 from Givaudan S.A. | 0.9 |
| | CAFS Microemulsion | 4 |

| | | |
|---|---|---|
| Part A: Components of part A were mixed with overhead mechanical stirrer for 30 minutes at 600 rpm. Part B was added to part A and stirred at 600 rpm for 15 minutes. Part C: Components of part C were mixed together with mechanical stirrer at 600 rpm for 30 minutes. Part A and part B were added to part C and mixed with overhead mechanical stirrer at 600 rpm for 30 minutes. Part D: Components of part D were mixed with magnetic stirrer at 200 rpm for 15 minutes. Part D was added to part A+B+C and then mixed for 30 minutes with mechanical stirrer at 600 rpm for 30 minutes. | | |

### Perfume Applications

### Application Example 9: Ethanol Based Perfume Rub in

### Ethanol based Perfume Formulation (F11) - Synthetic Example 3 (D25) containing Formulation

**TABLE 46**

| Component | % |
|---|---|
| Synthetic Example 3 | 0.3 |
| Fragrance Floral Fruity R13-5175 from Robertet, Inc. | 0.1 |
| Ethanol | q.s. to 100 |

0.3% D25 and 0.1% fragrance was mixed together for 5 minutes with a magnetic stirrer and then diluted with ethanol to reach 100%.

### Comparative Ethanol based Perfume Formulation (C16) - Synthetic Example 2 (D15) containing Formulation

**TABLE 47**

| Component | % |
|---|---|
| Synthetic Example 2 | 0.3 |
| Fragrance Floral Fruity R13-5175 from Robertet, Inc. | 0.1 |
| Ethanol | q.s. to 100 |

0.3% D15 and 0.1% fragrance was mixed together for 5 minutes with a magnetic stirrer and then diluted with ethanol to reach 100%.

### Comparative Ethanol based Perfume Formulation (C17) - Negative Control Formulation

**TABLE 48**

| Component | % |
|---|---|
| Fragrance Floral Fruity R13-5175 from Robertet, Inc. | 0.1 |
| Ethanol | q.s. to 100 |

| | |
|---|---|
| 0.1% fragrance was diluted with ethanol to reach 100%. | |

Perfume Application: 3 samples of single bleached Asian hair were used. 1 mL perfume was applied on 4 g hair tress and the perfume was rubbed thoroughly into hair. The hair tress was then dried under bonnet with cool air setting. The evaluation was done 0 hour and 5 hours after drying.

Evaluation: A panel study was conducted using 4 trained panelists. Each panelist was asked to smell coffee prior to and between smelling tresses to clear their nose. Then the panelist was asked to rank the hair samples based on strength of smell.

Results: All four panelists chose D25 treated hair > D15 treated hair > negative control for both 0 and 5 hours. This is consistent with the water based leave on conditioner result in D length effect section of application examples.

### Application Example 10: Ethanol Based Perfume Spray On

### Ethanol based Perfume Formulation (F12) - Synthetic Example 12 (MD73D'27M) containing Formulation

**TABLE 49**

| Component | % |
|---|---|
| Synthetic Example 12 | 10 |
| Perfume 192 from Givaudan S.A. | 10 |
| Ethanol | q.s. to 100 |

### Comparative Ethanol based Perfume Formulation (C18) - Synthetic Example 9 (D25C4) containing Formulation

**TABLE 50**

| Component | % |
|---|---|
| Synthetic Example 9 | 10 |
| Perfume 192 from Givaudan S.A. | 10 |
| Ethanol | q.s. to 100 |

For each formulation, silicone and fragrance was mixed with ethanol for 15 minutes with a magnetic stirrer to get a clear solution.

Perfume Application: 2 samples of single bleached Asian hair were used. 2 sprays of perfume on each side of 2 gram tress applied 0.15 gram of perfume. The hair tress was then dried under bonnet with cool air setting. The evaluation was done 0 hour and 5 hours after drying.

Evaluation: A panel study was conducted using 4 trained panelists. Each panelist was asked to smell coffee prior to and between smelling tresses to clear their nose. Then the panelist was asked to rank the hair samples based on strength of smell.

Results: All four panelists chose Synthetic Example 12 treated hair > D25C4 treated hair for both 0 and 5 hours.

### Application Example 10: 85% Ethanol 15% Water Based Perfume Spray On Ethanol/Water based Perfume Formulation (F13) - Synthetic Example 12 (MD73D'27M) containing Formulation

**TABLE 51**

| Component | % |
|---|---|
| Synthetic Example 12 | 10 |
| Perfume 192 from Givaudan S.A. | 10 |
| 85% Ethanol 15% Water | q.s. to 100 |

| | |
|---|---|
| 10% Synthetic Example 12 and 10% fragrance was mixed with 85% ethanol/15% water for 15 minutes with a magnetic stirred to get a clear perfume solution. | |

### Comparative Ethanol/Water based Perfume Formulation (C19) - Negative Control Formulation

**TABLE 52**

| Component | % |
|---|---|
| Perfume 192 from Givaudan S.A. | 10 |
| 85% Ethanol 15% Water | q.s. to 100 |

| | |
|---|---|
| 10% fragrance and 85% ethanol/15% water was mixed together to get a clear solution. | |

Perfume Application: 2 samples of single bleached Asian hair were used. 2 sprays of perfume on each side of 2 gram tress applied 0.15 gram of perfume. The hair tress was then dried under bonnet with cool air setting. The evaluation was done 1 hour, 4 hours, 24 hours and 4 days after drying.

Evaluation: A panel study was conducted using 5 trained panelists. Each panelist was asked to smell coffee prior to and between smelling tresses to clear their nose. Then the panelists were asked to rate the fragrance smell from 0 to 5. 0 is not noticeable and 5 is very strong.

Results: For 1 hour, all five panelists chose synthetic example 12 treated hair has stronger fragrance smell than negative control, but they couldn't differentiate the smell by scale because both smell were very strong. The 4 hour result is shown in Table 39, the scale difference between Synthetic Example 12 treated hair and negative control is 2 based on 0-5 scale, and is very significant. After 24 hours, the delta is still 2 and after 4 days, 3 panelists still noticed Synthetic Example 12 treated hair had obviously stronger fragrance smell.

### 6 Panelists Comparison of Synthetic Example 12 (MD73D'27M) containing Formulation F13 and Negative Control Formulation C19

**TABLE 53**

| Time after application | Synthetic Example 12 containing F13 Score | F13 Standard Deviation | Negative Control Formulation C19 Score | C19 Standard Deviation |
|---|---|---|---|---|
| 4 Hours | 3.4 | 0.55 | 1.4 | 0.55 |

### Textile Application

### Application Example 10: Dip in Treatment for Fabrics

### Dip in Treatment Formulation (F14)

**TABLE 54**

| Component | % |
|---|---|
| CAFS Microemulsion | 1.2 |
| Fragrance 6113578 from Bell Flavors & Fragrances Inc. | 0.1 |
| Water | q.s. to 100 |

| | |
|---|---|
| 1.2% CAFS microemulsion and 0.1% fragrance was mixed together for 5 minutes with a magnetic stirrer and then diluted with water to reach 100%. | |

### Comparative Formulation (C20) - Negative Control Formulation

**TABLE 55**

| Component | % |
|---|---|
| Negative Control Emulsion | 1.2 |
| Fragrance 6113578 from Bell Flavors & Fragrances Inc | 0.1 |
| Water | q.s. to 100 |

| | |
|---|---|
| 1.2% negative control emulsion and 0.1% fragrance was mixed together for 5 minutes with a magnetic stirrer and then diluted with water to reach 100%. | |

### Fabrics

### Bleached Mercerized Cotton Twill

### Cotton/polyester blend (50/50)

### Filament Nylon

### Non-woven polyester

Dip in Treatment: Each substrate was cut into 7" × 1" strips, and dipped in only the treating solution F14 or C20, hand squeezed, and bonnet dried using the low heat setting for 30 minutes. Samples were hung on a bench top rack and allowed to acclimate to ambient conditions for 1 hour. Evaluation was done over the next 2-3 hours and 24 hours later.

Evaluation: A panel study was conducted using 5 trained panelists. Each panelist were asked to smell coffee prior to and between smelling tresses to clear their nose. Each panelist smelled the tresses and ranked tresses in order of strength of fragrance smell.

Results: 5 of 5 panelists picked the carboxylic acid functional silicone containing dip in treatment formulation F14 over negative control dip in treatment formulation C20 for both 1-3 hours and 24 hours after acclimation.

### Effect of D Length

To study the effect of D length on fragrance retention effect, D15 side chain and D25, D50, D100, D200, D300, D500 end block carboxylic INX varieties were synthesized.

### Leave on Conditioner Formulation 1 (F15)

**TABLE 56**

| Component | % |
|---|---|
| CAFS Microemulsion | 1.2 |
| Fragrance Floral Fruity R13-5175 from Robertet, Inc. | 0.1 |
| Water | q.s. to 100 |

A 1.2% CAFS microemulsion and 0.1% fragrance were mixed together for 5 minutes with a magnetic stirrer and then diluted with water to reach 100%.

### Comparative Formulation 1 (C21) - Element 14 PDMS 100 containing formulation

**TABLE 57**

| Component | % |
|---|---|
| Element 14 PDMS 100^{∗} | 0.3 |
| Negative Control Emulsion | 1.2 |
| Fragrance Floral Fruity R13-5175 from Robertet, Inc. | 0.1 |
| Water | q.s. to 100 |

| | |
|---|---|
| ^{∗}Element 14 PDMS 100 is dimethicone with kinematic viscosity of 100 cst; It is available from Momentive. | |

### Comparative Formulation 2 (C22) - Synthetic Example 2 (D15) containing formulation

**TABLE 58**

| Component | % |
|---|---|
| Synthetic Example 2 | 0.3 |
| Negative Control Emulsion | 1.2 |
| Fragrance Floral Fruity R13-5175 from Robertet, Inc. | 0.1 |
| Water | q.s. to 100 |

### Comparative Formulation 3 (C23) - Synthetic Example 3 (D25) containing formulation

**TABLE 59**

| Component | % |
|---|---|
| Synthetic Example 3 | 0.3 |
| Negative Control Emulsion | 1.2 |
| Fragrance Floral Fruity R13-5175 from Robertet, Inc. | 0.1 |
| Water | q.s. to 100 |

### Comparative Formulation 4 (C24) - Synthetic Example 4 (D50) containing formulation

**TABLE 60**

| Component | % |
|---|---|
| Synthetic Example 4 | 0.3 |
| Negative Control Emulsion | 1.2 |
| Fragrance Floral Fruity R13-5175 from Robertet, Inc. | 0.1 |
| Water | q.s. to 100 |

### Comparative Formulation 5 (C25) - Synthetic Exmaple 5 (D200) containing formulation

**TABLE 61**

| Component | % |
|---|---|
| Synthetic Exmaple 5 | 0.3 |
| Negative Control Emulsion | 1.2 |
| Fragrance Floral Fruity R13-5175 from Robertet, Inc. | 0.1 |
| Water | q.s. to 100 |

### Comparative Formulation 6 (C26) - Synthetic Example 6 (D300) containing formulation

**TABLE 62**

| Component | % |
|---|---|
| Synthetic Example 6 | 0.3 |
| Negative Control Emulsion | 1.2 |
| Fragrance Floral Fruity R13-5175 from Robertet, Inc. | 0.1 |
| Water | q.s. to 100 |

### Comparative Formulation 7 (C27) - Synthetic Example 7 (D500) containing formulation

**TABLE 63**

| Component | % |
|---|---|
| Synthetic Example 7 | 0.3 |
| Negative Control Emulsion | 1.2 |
| Fragrance Floral Fruity R13-5175 from Robertet, Inc. | 0.1 |
| Water | q.s. to 100 |

A 0.3% Element 14 PDMS 100 or synthetic example 2, synthetic example 3, synthetic example 4, synthetic example 5, synthetic example 6, synthetic example 7, 1.2% negative control emulsion and 0.1% fragrance were mixed together for 5 minutes with magnetic stirrer and then diluted with water to reach 100%.

Leave-on Treatment: 8 samples of Virgin Latin hair were used. Each sample was dipped in only one of the leave on conditioner formulations F15, C21, C22, C23, C24, C25, C26 and C27 for 1 minute. The extra from each sample was squeezed out and the hair was blow dried at low heat for 10-15 minutes until fully dry. The evaluation was done after the samples stay in room condition open air 1-2 hours, 24 hours and 48 hours.

Evaluation: A panel study was conducted using 4-5 trained panelists. Each panelist was asked to smell coffee prior to and between smelling tresses to clear their nose. The hair samples were separated into 2 groups A and B. A group was treated by C21 (PDMS 100), C22 (D15), C23 (D25), C24 (D50), and B group was treated by F15 (D100), C25 (D200), C26 (D300) and C27 (D500). Each panelist smelled the tresses in A and B group and ranked tresses in order of strength of fragrance smell for each group. And then each panelist smelled the strongest smell tress from A and B group and chose the stronger smell one as the strongest smell tress of all hair samples.

### Results:

### Initial Smell

**TABLE 64**

| Rater | Group A Evaluation | Group B Evaluation | A vs. B |
|---|---|---|---|
| P1 | C23>C22>C24>C21 | F15>C26>C25>C27 | F15>C23 |
| P2 | C22>C23>C24>C21 | F15>C26>C25>C27 | F15>C22 |
| P3 | C23>C22>C24>C21 | F15>C25>C26>C27 | F15>C23 |
| P4 | C23>C22>C24>C21 | F15>C26>C25>C27 | F15>C23 |

### 24 Hours Smell

**TABLE 65**

| Rater | Group A Evaluation | Group B Evaluation | A vs. B |
|---|---|---|---|
| P1 | C23>C22>C24>C21 | F15>C26>C25>C27 | F15>C23 |
| P2 | C22>C23>C24>C21 | F15>C25>C26>C27 | F15>C22 |
| P3 | C23>C22>C24>C21 | F15>C26>C25>C27 | F15>C23 |
| P4 | C23>C24>C21>C22 | F15>C25>C26>C27 | F15>C23 |
| P5 | C23>C22>C24>C21 | F15>C26>C25>C27 | F15>C23 |

### 48 Hours Smell

**TABLE 66**

| Rater | Group A Evaluation | Group B Evaluation | A vs. B |
|---|---|---|---|
| P1 | C23>C22>C24>C21 | F15>C25>C26>C27 | F15>C23 |
| P2 | C21>C22>C24>C23 | F15>C27>C26>C25 | F15>C21 |
| P3 | C23>C22>C24>C21 | F15>C25>C26>C27 | F15>C23 |
| P4 | C22>C23>C24>C21 | F15>C27>C25>C26 | F15>C22 |
| P5 | C23>C22>C24>C21 | F15>C26>C25>C27 | F15>C23 |

The results of the evaluations in Tables 64, 65 and 66 clearly indicate the personal care formulations containing the preparations of Synthetic Examples 1 and 11 provide better fragrance retention than control formulation C21.

### Effect of D length when End Block and Pendant Carbon Chain Length are 4

### Carboxylic Acid Functional Silicone (CAFS) Emulsion II

**TABLE 67**

| | | Emulsion E1 | Emulsion E2 |
|---|---|---|---|
| | Chemical Name | % | % |
| A | Synthetic Example 9 (component (a)) | 25 | - |
| | Synthetic Example 11 (component (a)) | - | 25 |
| | Dipropylene Glycol (surfactant b) | 5 | 5 |
| | Trideceth-6 (surfactant b) | 18.8 | 18.8 |
| B | Sodium Chloride | 1 | 1 |
| | Water (c) | 40.3 | 40.3 |
| C | Sodium Hydroxide | 0.2 | 0.2 |
| | Water (c) | q.s. to 100 | q.s. to 100 |

1. Part A was mixed by mixing C4 CAFS component (a) and the two (b) surfactants together and heated to 50 °C, the mixing was continued for 15 minutes at 300 rpm using an overhead stirrer.
2. Part B: A solution of sodium chloride in water was mixed, and, added in four parts with mixing to part A until completely incorporated.
3. Part C: A solution of sodium hydroxide in water was mixed and then added to the A+B mixture. The mixing speed was reduced after 15 minutes and the emulsion was allowed to cool to room temperature. The pH was checked and adjusted by citric acid to 6.5-7.

### Carboxylic Acid Functional Silicone (CAFS) Emulsion III

**TABLE 68**

| | Emulsion E3 | Emulsion E4 | Emulsion E5 |
|---|---|---|---|
| Chemical Name | % | % | % |
| Synthetic Example 9 | 25 | - | - |
| Synthetic Example 11 | - | 25 | - |
| Synthetic Example 12 | - | - | 25 |
| Isolaureth-10 | 12 | 12 | 12 |
| Water | q.s. to 100 | q.s. to 100 | q.s. to 100 |

CAFS (C4 version) and surfactant isolaureth-10 was mixed for 15 minutes at 300 rpm using an overhead stirrer. Water was added to the mixture and the mixing continued for another 15 minutes. Sodium hydroxide was then used to adjust the pH to 6.5-7.

### Leave on Conditioner Formulation 1 (F16) - Synthetic Example 11 (D100 C4) CAFS & Floral Fruity containing formulation

**TABLE 69**

| Component | % |
|---|---|
| Emulsion E2 | 1.2 |
| Fragrance Floral Fruity R13-5175 from Robertet, Inc. | 0.1 |
| Water | q.s. to 100 |

### Leave on Conditioner Formulation 2 (F17) - Synthetic Example 9 (D25 C4) CAFS & Floral Fruity containing formulation

**TABLE 70**

| Component | % |
|---|---|
| Emulsion E1 | 1.2 |
| Fragrance Floral Fruity R13-5175 from Robertet, Inc. | 0.1 |
| Water | q.s. to 100 |

### Comparative Formulation 1 (C28) - Element 14 PDMS 100 & Floral Fruity containing Formulation

**TABLE 71**

| Component | % |
|---|---|
| Element 14 PDMS 100^{∗} | 0.3 |
| Negative Control Emulsion | 1.2 |
| Fragrance Floral Fruity R13-5175 from Robertet, Inc. | 0.1 |
| Water | q.s. to 100 |

| | |
|---|---|
| ^{∗}Element 14 PDMS 100 is a dimethicone with a kinematic viscosity of 100 cst (Momentive Performance Materials Inc.). | |

### Leave on Conditioner Formulation 3 (F18) - Synthetic Example 11 (D100 C4) CAFS & Perfume 192 containing Formulation

**TABLE 72**

| Component | % |
|---|---|
| Emulsion E2 | 1.2 |
| Perfume 192 from Givaudan S.A. | 0.1 |
| Water | q.s. to 100 |

### Leave on Conditioner Formulation 4 (F19) - Synthetic Example 9 (D25 C4) CAFS & Perfume 192 containing Formulation

**TABLE 73**

| Component | % |
|---|---|
| Emulsion E1 | 1.2 |
| Perfume 192 from Givaudan S.A. | 0.1 |
| Water | q.s. to 100 |

### Comparative Formulation 2 (C29) - Element 14 PDMS 100 & Perfume 192 containing Formulation

**TABLE 74**

| Component | % |
|---|---|
| Element 14 PDMS 100^{∗} | 0.3 |
| Negative Control Emulsion | 1.2 |
| Perfume 192 from Givaudan S.A. | 0.1 |
| Water | q.s. to 100 |

### Leave on Conditioner Formulation 5 (F20) - Synthetic Example 11 (D100 C4) & Floral Fruity containing Formulation

**TABLE 75**

| Component | % |
|---|---|
| Emulsion E4 | 1.2 |
| Fragrance Floral Fruity R13-5175 from Robertet, Inc. | 0.1 |
| Water | q.s. to 100 |

### Leave on Conditioner Formulation 6 (F21) - Synthetic Example 9 (D25 C4) & Floral Fruity containing Formulation

**TABLE 76**

| Component | % |
|---|---|
| Emulsion E3 | 1.2 |
| Fragrance Floral Fruity R13-5175 from Robertet, Inc. | 0.1 |
| Water | q.s. to 100 |

### Leave on Conditioner Formulation 7 (F22) - Synthetic Example 12 (MD73D'27M) & Perfume 192 containing Formulation

**TABLE 77**

| Component | % |
|---|---|
| Emulsion E5 | 1.2 |
| Perfume 192 from Robertet, Inc. | 0.1 |
| Water | q.s. to 100 |

### Leave on Conditioner Formulation 8 (F23) - Synthetic Example 9 (D25 C4) & Perfume 192 containing Formulation

**TABLE 78**

| Component | % |
|---|---|
| Emulsion E3 | 1.2 |
| Perfume 192 from Robertet, Inc. | 0.1 |
| Water | q.s. to 100 |

### Leave on Conditioner Formulation 9 (F24) - Synthetic Example 12 (MD73D'27M) & Connection 17 containing Formulation

**TABLE 79**

| Component | % |
|---|---|
| Emulsion E5 | 1.2 |
| Connection 17 from Givaudan S.A. | 0.1 |
| Water | q.s. to 100 |

### Leave on Conditioner Formulation 10 (F25) - Synthetic Example 9 (D25 C4) & Connection 17 containing Formulation

**TABLE 80**

| Component | % |
|---|---|
| Emulsion E3 | 1.2 |
| Connection 17 from Givaudan S.A. | 0.1 |
| Water | q.s. to 100 |

A 1.2% CAFS emulsion or CAFS Emulsion III and 0.1% fragrance were mixed together for 5 minutes with a magnetic stirrer and then diluted with water to reach 100%. A 0.3% Element 14 PDMS 100, 1.2% negative control emulsion and 0.1% fragrance were mixed together for 5 minutes with magnetic stirrer and then diluted with water to reach 100%.

Leave-on Treatment: Samples of Virgin Latin hair were used. Each sample was dipped in only one of the leave on conditioner formulations for 1 minute. The extra from each sample was squeezed out and the hair was blow dried at low heat for 10-15 minutes until fully dry. The evaluation was done after the samples stay in room condition for 24 hours.

Evaluation: A panel study was conducted using 5 trained panelists. Each panelist was asked to smell coffee prior to and between smelling tresses to clear their nose. Then the panelists were asked to rate the fragrance smell from 0 to 5. 0 is not noticeable and 5 is very strong.

### Results:

### Synthetic Example 11 (D 100 C4) vs PDMS for Perfume 192 & Floral Fruity with Emulsion Package I

### Floral Fruity: Synthetic Example 11 (D100 C4) Formulation F16 and PDMS Formulation C28

### Perfume 192: Synthetic Example 11 (D100 C4) Formulation F18 and PDMS Formulation C29

**TABLE 81**

| Perfume | D100 C4 containing Formulation Score | Standard Deviation | PDMS containing Formulation Score | Standard Deviation |
|---|---|---|---|---|
| Floral Fruity | 3.4 | 0.55 | 2.2 | 0.45 |
| Perfume 192 | 2.4 | 0.55 | 1.4 | 0.55 |

### Synthetic Example 9 (D25 C4) vs PDMS for Perfume 192 & Floral Fruity with Emulsion Package I

### Floral Fruity: Synthetic Example 9 (D25 C4) Formulation F17 and PDMS Formulation C28

### Perfume 192: Synthetic Example 9 (D25 C4) Formulation F19 and PDMS Formulation C29

**TABLE 82**

| Perfume | D25 C4 containing Formulation Score | Standard Deviation | PDMS containing Formulation Score | Standard Deviation |
|---|---|---|---|---|
| Floral Fruity | 1.6 | 0.55 | 1 | 0 |
| Perfume 192 | 1.8 | 0.84 | 1.4 | 0.55 |

### Synthetic Example 11 (D100 C4) vs Synthetic Example 9 (D25 C4) for Floral Fruity

### Floral Fruity: Synthetic Example 11 (D100 C4) Formulation F20 and Synthetic Example 9 (D25 C4) Formulation F21

**TABLE 83**

| Perfume | D100 C4 containing Formulation Score | Standard Deviation | Synthetic Example 9 (D25 C4) containing Formulation Score | Standard Deviation |
|---|---|---|---|---|
| Floral Fruity | 3.6 | 0.55 | 1.8 | 0.45 |

### Synthetic Example 12 (MD73D'27M) vs Synthetic Example 9 (D25 C4) for Perfume 192 & Connection 17 with Emulsion Package II

### Perfume 192: Synthetic Example 12 (MD73D'27M) Formulation F22 and Synthetic Example 9 (D25 C4) Formulation F23

### Connection 17: Synthetic Example 12 (MD73D'27M) Formulation F24 and Synthetic Example 9 (D25 C4) Formulation F25

**TABLE 84**

| Perfume | Synthetic Example 12 (MD73D'27M) containing Formulation Score | Standard Deviation | Synthetic Example 9 (D25 C4) containing Formulation Score | Standard Deviation |
|---|---|---|---|---|
| Perfume 192 | 2.4 | 0.55 | 1.8 | 0.84 |
| Connection 17 | 3.4 | 0.55 | 2.4 | 0.55 |

From these results, we can see the fragrance retention capacity of CAFS C4 version has similar trend as that of CAFS C10 version. Synthetic Example 11 (D100 C4) is better than Synthetic Example 9 (D25 C4), with end block carboxylic D100 slightly better than pendant chain D100. Considering pendant chain D100 has much more carboxylic groups than end block carboxylic D100 version, end block carboxylic works much more efficiently than pendant carboxylic in fragrance retention.

## Claims

1. A fragrance delivery composition comprising:
(a) a fragrance longevity enhancing amount of 0.01 wt% to 60 wt% of a neutralized acid-functional silicone of the general formula (I):
MₐMⁱ_{b}D_{c}Dⁱ_{d}TₑTⁱ_{f}Q_{g} (I)
wherein
M = R¹R²R³SiO_{1/2};
Mⁱ = R⁴R⁵RⁱSiO_{1/2};
D = R⁶R⁷SiO_{2/2};
Dⁱ = R⁸RⁱSiO_{2/2};
T = R⁹SiO_{3/2};
Tⁱ = RⁱSiO_{3/2};
Q = SiO_{4/2};
where
R¹, R², R³, R⁴, R⁵, R⁶, R⁷, R⁸, and R⁹ are each independently selected from the group of monovalent hydrocarbon radicals having from 1 to 60 carbon atoms,
Rⁱ is a monovalent hydrocarbon radical having from 2 to 60 carbon atoms and containing one or more acid functionality groups selected from the group consisting of carboxylic acid-, phosphonic acid- and sulfonic acid-containing groups, their salts and combinations thereof, and
subscripts a, b, c, d, e, f, and g are independently zero or a positive number subject to the limitations b + d + f ≥ 1 and a + b + c + d + e + f < 1000;
(b) 0.01 wt% to 10 wt% of a fragrance; and,
(c) an emulsion, suspension or organic solvent solution forming amount of water and/or organic solvent

2. The fragrance delivery composition of Claim 1 in the form of an aqueous or organic solvent emulsion, an aqueous or organic solvent suspension, or an organic solvent solution or miscible mixture of organic solvent and water.

3. The fragrance delivery composition of Claim 1 wherein in neutralized acid-functional silicone (a), subscripts a, d, e, f, and g are 0, subscript b is 2, subscript c is from 0 to 300 and each Rⁱ is independently of the general formula (II): 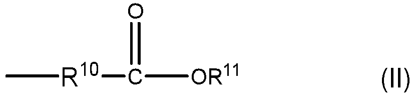 wherein
R¹⁰ is a divalent hydrocarbon moiety selected from alkyl, aryl, or alkylaryl containing from 1 to 60 carbon atoms, and
R¹¹ is selected from hydrogen, alkali metal, an aminium group or a quaternized nitrogen group.

4. The fragrance delivery composition of Claim 1 wherein neutralized acid-functional silicone (a) has a pH of from 5 to 9.

5. The fragrance delivery composition of Claim 1 further comprising an emulsifier or suspending agent (d) which is selected from the group consisting of a nonionic surfactant, ionic surfactant, anionic surfactant, cationic surfactant, amphoteric surfactant, zwitterionic surfacant and combinations thereof, wherein the suspending agent (d) is preferably selected from the group consisting of hexylbenzenesulfonic acid, octylbenzenesulfonic acid, decylbenzylsulfonic acid, dodecylbenzenesulfonic acid, cetylbenzenesulfonic acid, myristylbenzenesulfonic acid and the salts of said acids; octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow trimethylammonium hydroxide, coconut oil trimethylammonium hydroxide, ethylene oxide adduct of diethylene glycol trimethyl nonanol, polypropylene glycol, polyethylene glycol, polyoxyalkylene sorbitan ester, polyoxyalkylene alkyl ester, polyoxyalkylene alkyl phenol, polyoxyalkylene alkyl ether and combinations thereof.

6. The fragrance delivery composition of Claim 1 wherein the at least one fragrance compound (b) is obtained from a source selected from the group consisting of essential oils, flower oils, natural extracts from resins, gums, balsams, beans, mosses, plants, ambergris, musk, synthetic aromatic materials and combinations thereof; or wherein the at least one fragrance compound (b) has a scent that is selected from the group consisting of citrus, floral, spicy, lavender, woody, mossy, oriental, herbal, leather-tobacco, aldehydic groups and combinations thereof.

7. The fragrance delivery composition of Claim 1 wherein the dispersed oil phase of the aqueous emulsion or suspension has a diameter of from 1 to 100 nm.

8. The fragrance delivery composition of Claim 1 wherein acid-functional silicone (I) is of the general formula (III): 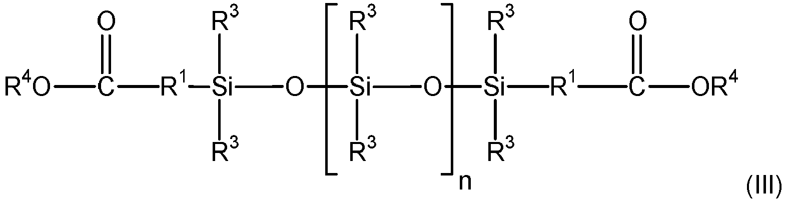 where the subscript n is from 0 to 300, R³ is a monovalent hydrocarbon radical with 1-6 carbon atoms, R¹ is a divalent moiety selected from alkyl, aryl or alkylaryl containing up to 30 carbon atoms, and R⁴ is selected from hydrogen, alkali metal, aminium group or quaternized nitrogen group.

9. A process of making the fragrance delivery composition of any preceding claim comprising combining constituents (a), (b) and (c) as defined under emulsion-, suspension- or organic solvent solution-forming conditions.

10. The process of Claim 9 wherein the step of combining comprises adding the components (a)-(c) of the aqueous emulsion or suspension simultaneously.

11. The process of Claim 9 wherein the step of combining comprises adding the neutralized acid-functional silicone (a) and at least one fragrance compound (b) simultaneously.

12. The process of Claim 9 wherein the step of combining comprises adding the components (a) and (c) of the aqueous emulsion or suspension and at least one fragrance compound (b) separately in a gel system such that the component (a) and (b) are suspended in the gel system.

13. A personal care formulation comprising the fragrance delivery composition of Claim 1, wherein the personal care formulation is preferably selected from the group consisting of deodorants, antiperspirants, antiperspirant/deodorants, shaving products, skin lotions, moisturizers, toners, bath products, cleansing products, shampoos, conditioners, mousses, styling gels, hair sprays, hair dyes, hair color products, hair bleaches, waving products, hair straighteners, nail polish, nail polish remover, nail creams and lotions, cuticle softeners, sunscreen, insect repellent and anti-aging products, lipsticks, foundations, face powders, eye liners, eye shadows, blushes, makeup, pet grooming products and mascaras, and more preferably selected from the group consisting of shampoo, conditioner, hair serum, skin cream, and skin serum.

14. A fabric having applied thereto the fragrance delivery composition of Claim 1, wherein the fabric is preferably selected from the group consisting of synthetic textiles, natural textiles and blends thereof.

15. A home care formulation comprising the fragrance delivery composition of Claim 1, wherein the home care formulation is preferably selected from the group consisting of laundry detergent and fabric softener, dishwashing liquids, wood and furniture polish, floor polish, tub and tile cleaners, toilet bowl cleaners, hard surface cleaners, window cleaners, antifog agents, drain cleaners, auto-dishwashing detergents and sheeting agents, carpet cleaners, prewash spotters, rust cleaners, automotive care products, leather cleaners, leather conditioners, room air fresheners, odor maskers and scale removers.

## Patentansprüche

1. Duftfreisetzungszusammensetzung, aufweisend:
(a) eine Menge von 0,01 Gew.-% bis 60 Gew.-% zur Verbesserung der Langlebigkeit eines Dufts aus einem funktionellen Neutralsäuresilikon der allgemeinen Formel (I):
MₐMⁱ_{b}Dⁱ_{c}Dⁱ_{d}TₑTⁱfQ_{g} (I)
wobei
M = R¹R²R³SiO_{1/2};
M' = R⁴R⁵RⁱSiO_{1/2}
D = R⁶R⁷SiO_{2/2};
Dⁱ = R⁸RiSiO_{2/2};
T = R⁹SiO_{3/2};
Tⁱ = RⁱSiO_{3/2} ;
Q = SiO_{4/2};
wobei
R¹, R², R³, R⁴, R⁵, R⁶, R⁷, R⁸ und R⁹ jeweils unabhängig ausgewählt sind aus der Gruppe einwertiger Kohlenwasserstoffreste mit 1 bis 60 Kohlenstoffatomen,
Rⁱ ein einwertiger Kohlenwasserstoffrest mit 2 bis 60 Kohlenstoffatomen ist und eine oder mehr funktionellen Säuregruppen enthält, die ausgewählt sind aus der Gruppe bestehend aus Carbonsäure-, Phosphonsäure- und Sulfonsäure-haltigen Gruppen, deren Salze und Kombinationen dieser, und
die Tieferstellungen a, b, c, d, e, f und g unabhängig null oder eine positive Zahl sind, mit den Beschränkungen b +d +f ≥ 1 und a +b +c+ d+ e +f < 1000;
(b) 0,01 Gew.-% bis 10 Gew.-% eines Dufts; und
(c) eine Emulsion, Suspension oder eine organische Lösemittellösung aus Wasser und/oder einem organischen Lösungsmittel.

2. Duftfreisetzungszusammensetzung nach Anspruch 1 in Form einer wässrigen oder organischen Lösungsmittelemulsion, einer wässrigen oder organischen Lösungsmittelsuspension oder einer organischen Lösungsmittellösung oder einem mischbaren Gemisch aus organischem Lösungsmittel und Wasser.

3. Duftfreisetzungszusammensetzung nach Anspruch 1, wobei in dem funktionellen Neutralsäuresilikon (a) die Tieferstellungen a, d, e, f und g = 0 sind, die Tieferstellung b = 2 ist, die Tieferstellung c = 0 bis 300 ist, und jedes Rⁱ unabhängig die allgemeine Formel (II) hat: 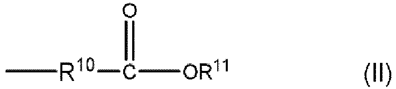 wobei
R¹⁰ ein zweiwertiger Kohlenwasserstoffrest ist, der ausgewählt ist aus Alkyl, Aryl oder Alkylaryl, und 1 bis 60 Kohlenstoffatomen enthält, und
R¹¹ ausgewählt ist aus Wasserstoff, einem Alkalimetall, einer Aminiumgruppe oder einer vierwertigen Stickstoffgruppe.

4. Duftfreisetzungszusammensetzung nach Anspruch 1, wobei das funktionelle Neutralsäuresilikon (a) einen pH-Wert von 5 bis 9 hat.

5. Duftfreisetzungszusammensetzung nach Anspruch 1, ferner aufweisend ein Emulgator- oder Suspensionsmittel (d), das ausgewählt ist aus der Gruppe bestehend aus einem nicht-ionischen Tensid, einem ionischen Tensid, einem anionischen Tensid, einem kationischen Tensid, einem amphoterischen Tensid, einem zwitterionischen Tensid und Kombinationen dieser, wobei das Suspensionsmittel (d) bevorzugt ausgewählt ist aus der Gruppe bestehend aus Hexylbenzensulfonsäure, Octylbenzensulfonsäure, Decylbenzylsulfonsäure, Dodecylbenzensulfonsäure, Cetylbenzensulfonsäure, Myristylbenzensulfonsäure und den Salzen dieser Säuren; Octyltrimethylammoniumhydoxid, Dodecyltrimethylammoniumhydroxid, Hexadecyltrimethylammoniumhydroxid, Octyldimethylbenzylammoniumhydoxid, Decyldiemthylbenzylammoniumhydroxyddioctadecyldiemthlyammoniumhydrox id, Dioctadecyldiemthylammoniumhydroxid, Rindertalgtrimethylammoniumhydoxid, Kokosöltrimethylammoniumhydroxid, ein Ethylenoxidaddukt aus Diethylglykoltrimethylnonanol, Polypropylenglykol, Polyethylenglykol, Polyoxyalkylensorbitanesther, Polyoxyalkylenalkylester, Polyoxyalkylenalkylphenol, Polyoxyalkylenalkylether und Kombinationen dieser.

6. Duftfreisetzungszusammensetzung nach Anspruch 1, wobei der mindestens eine Duftbestandteil (b) erhalten wird aus einer Quelle ausgewählt aus der Gruppe bestehend aus etherischem Öl, Blütenölen, natürlichen Extrakten aus Harzen, Gummis, Balsamharzen, Bohnen, Moosen, Pflanzen, Ambra, Moschus, synthetischen Aromastoffen und Kombinationen dieser; oder wobei der mindestens eine Duftbestandteil (b) einen Geruch hat, der ausgewählt ist aus der Gruppe bestehend aus Zitrus-, Blumen-, Gewürz-, Lavendel-, Holz-, Moos-, Oriental-, Kräuter-, Leder-Tabak- Duft, Aldehydlgruppen und Kombinationen dieser.

7. Duftfreisetzungszusammensetzung nach Anspruch 1, wobei die dispergierte Ölphase der wässrigen Emulsion oder Suspension einen Durchmesser von 1 bis 100 nm hat.

8. Duftfreisetzungszusammensetzung nach Anspruch 1, wobei das säurefunktionelle Silikon (I) eine mit der allgemeinen Formel (III) ist: 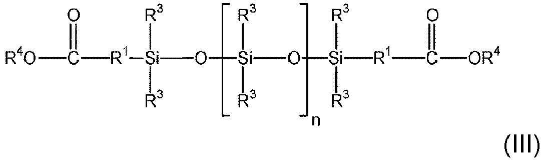 wobei die Tieferstellung n = 0 bis 300 beträgt, R3 ein einwertiger Kohlenwasserstoffrest mit 1 bis 6 Kohlenstoffatomen ist, R1 ein zweiwertiger Rest ist, der ausgewählt ist aus Akyl, Aryl oder Alkylaryl mit bis zu 30 Kohlenstoffatomen, und R4 ausgewählt ist aus Wasserstoff, einem Alkalimetall, einer Aminiumgruppe oder einer vierwertigen Stickstoffgruppe.

9. Verfahren zur Herstellung der Duftfreisetzungszusammensetzung nach einem der vorstehenden Ansprüche, umfassend das Kombinieren der Bestandteile (a), (b) und (c) wie unter Bedingungen zur Bildung von Emulsionen, Suspensionen oder organischen Lösungsmittellösungen definiert.

10. Verfahren zur Herstellung nach Anspruch 9, wobei der Schritt zum Kombinieren die zeitgleiche Hinzugabe der Bestandteile (a)-(c) der wässrigen Lösung oder der Suspension umfasst.

11. Verfahren zur Herstellung nach Anspruch 9, wobei der Schritt zum Kombinieren die zeitgleiche Hinzugabe des funktionellen Neutralsäuresilikons (a) und mindestens einem Duftbestandteil (b) umfasst.

12. Verfahren nach Anspruch 9, wobei der Schritt des Kombinierens die Hinzugabe der Bestandteile (a) und (c) der wässrigen Lösung oder Suspension und mindestens einen Duftbestandteil (b) in ein Gelsystem derart umfasst, dass Bestandteil (a) und (b) im Gelsystem suspendiert sind.

13. Körperpflegezusammensetzung, aufweisend die Duftfreisetzungszusammensetzung nach Anspruch 1, wobei diese Körperpflegeformulierung bevorzugt ausgewählt ist aus der Gruppe bestehend aus Deodorants, Antiperspiranten/Deodorants, Rasierprodukten, Hautlotionen, feuchtigkeitsspendenden Mitteln, Tonern, Badprodukten, Reinigungsprodukten, Shampoos, Conditionern, Mousses, Styling-Gels, Haarsprays, Haartönungen, Haarfärbeprodukten, Haaraufhellern, Wellungsprodukten, Glättungsprodukten, Nagellack, Nagellackentferner, Nagelcremes und Lotionen, Nagelhautweichmacher, Sonnencreme, Insektenspray und Anti-Aging-Produkten, Lippenstift, Foundations, Gesichtspudern, Eyelinern, Lidschatten, Rouge, Makeup, Haustierpflegeprodukte und Mascaras, und ferner ausgewählt ist aus der Gruppe bestehend aus Shampoo, Conditioner, Haarserum, Hautcreme und Hautserum.

14. Textil mit der darauf aufgetragenen Duftfreisetzungszusammensetzung nach Anspruch 1, wobei das Textil bevorzugt ausgewählt ist aus der Gruppe bestehend aus synthetischen Textilien, natürlichen Textilien und Mischungen dieser.

15. Haushaltsreinigerformulierung, aufweisend die Duftfreisetzungszusammensetzung nach Anspruch 1, wobei die Haushaltsreinigerformulierung bevorzugt ausgewählt ist aus der Gruppe bestehend aus Waschmittel und Weichspüler, Geschirrspülmitteln, Holz- und Möbelpolitur, Bodenpolitur, Badreinigern, WC-Reinigern, Antibeschlagmitteln, Abflussreinigern, Geschirrspülmaschinenmitteln und Versiegelungsmitteln, Teppichreinigungsmitteln, Fleckenspray, Rostentferner, Autopflegeprodukten, Lederreinigungsprodukten, Lederpflegemitteln, Raumlufterfrischern, Duftübertünchern und Rußentfernern.

## Revendications

1. Composition de distribution de parfum, la composition comprenant :
(a) une quantité d'amélioration de la durée du parfum, comprise entre 0,01 % en poids et 60 % en poids, d'une silicone à fonctionnalité acide neutralisée représentée par la formule générale (I) :
MₐMⁱ_{b}D_{c}Dⁱ_{d}TₑTⁱ_{f}Q_{g} (I)
dans laquelle
M = R¹R²R³SiO_{1/2} ;
M' = R⁴R⁵RⁱSiO_{1/2} ;
D = R⁶R⁷SiO_{2/2} ;
Dⁱ = R⁸RⁱSiO_{2/2} ;
T = R⁹SiO_{3/2} ;
Tⁱ = RⁱSiO_{3/2} ;
Q = SiO_{4/2} ;
où
R¹, R², R³, R⁴, R⁵, R⁶, R⁷, R⁸ et R⁹ sont chacun choisis indépendamment dans le groupe constitué par des radicaux hydrocarbures monovalents comportant 1 à 60 atomes de carbone,
Rⁱ est un radical hydrocarbure monovalent comportant 2 à 60 atomes de carbone et contenant un ou plusieurs groupements à fonctionnalité acide choisis dans le groupe constitué par des groupements contenant de l'acide carboxylique, de l'acide phosphonique et de l'acide sulfonique, leurs sels et leurs combinaisons, et
les indices a, b, c, d, e, f et g sont indépendamment zéro ou un nombre entier positif soumis aux limitations b + d + f ≥ 1 et a + b + c + d + e + f < 1 000 ;
(b) 0,01 % en poids à 10 % en poids d'un parfum ; et
(c) une quantité, formant une émulsion, une suspension ou une solution de solvant organique, d'eau et/ou d'un solvant organique.

2. La composition de distribution de parfum de la Revendication 1, sous forme d'une émulsion aqueuse ou de solvant organique, d'une suspension aqueuse ou de solvant organique ou d'une solution de solvant organique ou d'un mélange miscible de solvant organique et d'eau.

3. La composition de distribution de parfum de la Revendication 1, dans laquelle, dans la silicone à fonctionnalité acide neutralisée (a), les indices a, d, e, f et g sont 0, l'indice b est 2, l'indice c est compris entre 0 et 300 et chaque Rⁱ est représenté indépendamment par la formule générale (II) : 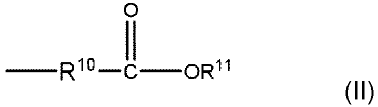 dans laquelle
R¹⁰ est une fraction hydrocarbure divalente choisi parmi un alkyle, un aryle ou un alkylaryle contenant 1 à 60 atomes de carbone, et
R¹¹ est choisi parmi l'hydrogène, un métal alcalin, un groupe aminium ou un groupe azote quaternarisé.

4. La composition de distribution de parfum de la Revendication 1, dans laquelle la silicone à fonctionnalité acide neutralisée (a) présente un pH compris entre 5 et 9.

5. La composition de distribution de parfum de la Revendication 1, comprenant en outre un agent émulsifiant ou de suspension (d) choisi dans le groupe constitué par un tensioactif non ionique, un tensioactif ionique, un tensioactif anionique, un tensioactif cationique, un tensioactif amphotère, un tensioactif zwitterionique et leurs combinaisons, l'agent de suspension (d) étant choisi de préférence dans le groupe constitué par l'acide héxylbenzénosulfonique, l'acide octylbenzénosulfonique, l'acide décylbenzylsulfonique, l'acide dodécylbenzénosulfonique, l'acide cétylbenzénosulfonique, l'acide myristylbenzénosulfonique et les sels desdits acides ; l'hydroxyde d'octyltriméthylammonium, l'hydroxyde de dodécyltriméthylammonium, l'hydroxyde d'hexadécyltrimethylammonium, l'hydroxyde d'octyldiméthylbenzylammonium, l'hydroxyde de décyldiméthylbenzylammonium, l'hydroxyde de dioctadécyldiméthylammonium, l'hydroxyde de triméthylammonium de suif de bœuf, l'hydroxyde de triméthylammonium d'huile de noix de coco, un adduit d'oxyde d'éthylène du diéthylène glycol triméthyl nonanol, le polypropylène glycol, le polyéthylène glycol, un ester de sorbitan de polyoxyalkylène, un ester d'alkyle de polyoxyalkylène, un alkyl phénol de polyoxyalkylène, un alkyl éther de polyoxyalkylène et leurs combinaisons.

6. La composition de distribution de parfum de la Revendication 1, dans laquelle le ou les composés de parfum (b) sont obtenus à partir d'une source choisie dans le groupe constitué par des huiles essentielles, des huiles florales, des extraits naturels de résines, gommes, haricots, mousses, plantes, ambre gris, musc naturel, des matières aromatiques synthétiques et leurs combinaisons ; ou dans laquelle le ou les composés de parfum (b) présentent une odeur choisie dans le groupe constitué par l'odeur citrique, florale, épicée, de lavande, boisée, de mousse, orientale, d'herbes, de cuire-tabac, de groupes aldéhydiques et leurs combinaisons.

7. La composition de distribution de parfum de la Revendication 1, dans laquelle la phase huile dispersée de l'émulsion ou suspension aqueuse présente un diamètre compris entre 1 et 100 nm.

8. La composition de distribution de parfum de la Revendication 1, dans laquelle la silicone à fonctionnalité acide (I) est représentée par la formule générale (III) : 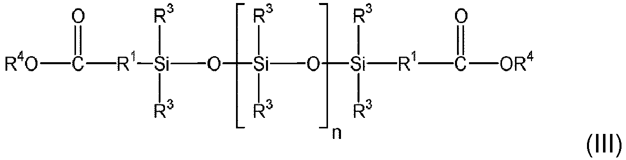 dans laquelle l'indice n est compris entre 0 et 300, R³ est un radical hydrocarbure monovalent contenant 1-6 atomes de carbone, R¹ est une fraction divalente choisie parmi l'alkyle, l'aryle ou l'alkylaryle contenant jusqu'à 30 atomes de carbone, et R⁴ est choisi parmi l'hydrogène, un métal alcalin, un groupe aminium ou un groupe azote quaternarisé.

9. Procédé de préparation de la composition de distribution de parfum de l'une quelconque des revendications précédentes, le procédé comprenant la combinaison des constituants (a), (b) et (c) tels que définis, sous conditions de formation d'émulsion, de suspension ou de solution de solvant organique.

10. Le procédé de la Revendication 9, dans lequel l'étape de combinaison comprend l'addition des constituants (a)-(c) de l'émulsion ou suspension aqueuse simultanément.

11. Le procédé de la Revendication 9, dans lequel l'étape de combinaison comprend l'addition de la silicone à fonctionnalité acide neutralisée (a) et d'au moins un composé de parfum (b) simultanément.

12. Le procédé de la Revendication 9, dans lequel l'étape de combinaison comprend l'addition des constituants (a) et (c) de l'émulsion ou suspension aqueuse et d'au moins un composé de parfum (b) séparément dans un système de gel, de sorte que les constituants (a) et (b) soient suspendus dans le système de gel.

13. Formulation de soins personnels comprenant la composition de distribution de parfum de la Revendication 1, la formulation de soins personnels étant choisie de préférence dans le groupe constitué par les déodorants, les antitranspirants, les antitranspirants/déodorants, les produits de rasage, les lotions pour la peau, les crèmes hydratantes, les lotions toniques, les produits pour le bain, les produits de nettoyage, les shampooings, les après-shampooing, les mousses, les gels coiffants, les laques à cheveux, les teintures pour les cheveux, les produits de coloration capillaire, les décolorants des cheveux, les produits d'ondulation, les produits de lissage, les vernis à ongles, les dissolvants, les crèmes et lotions pour les ongles, les adoucisseurs de cuticule, les écrans solaires, les répulsifs à insectes et les produits anti-âge, les rouges à lèvres, les fonds de teint, les poudres pour le visage, les crayons pour les yeux, les fards à paupières, les blush, le maquillage, les produits de toilettage et les mascaras, et de préférence encore choisie dans le groupe constitué par le shampooing, l'après-shampooing, le sérum pour les cheveux, la crème pour la peau et le sérum pour la peau.

14. Tissu comportant la composition de distribution de parfum de la Revendication 1 appliquée au tissu, le tissu étant choisi de préférence dans le groupe constitué par les textiles synthétiques, les textiles naturels et leurs mélanges.

15. Formulation d'entretien de la maison comprenant la composition de distribution de parfum de la Revendication 1, la formulation d'entretien de la maison étant choisie de préférence dans le groupe constitué par les lessives et les assouplissants, les liquides vaisselle, les cires pour bois et meubles, les cires pour le sol, les nettoyants de baignoire et de carrelage, les nettoyants des cuvettes des toilettes, les nettoyants de surfaces dures, les nettoyants de fenêtres, les agents antibuée, les nettoyants des canalisations, les détergents pour lave-vaisselle et les agents pour draps, les nettoyants de tapis, les détacheurs prélavage, les nettoyants de rouille, les produits d'entretien de la voiture, les nettoyants de cuir, les assouplissants de cuir, les désodorisants d'air, les masqueurs d'odeur et les produits antitartre.
